# EUROPEAN PATENT APPLICATION

(11) **EP 4 268 583 A1**
(43) Date of publication of application: **01.11.2023**
(21) Application number: 21915286.5
(22) Date of filing: 27.12.2021
(51) Int. Cl.: A01H 5/00, A01H 1/00, A01H 4/00, A01H 6/46, C12N 5/10, C12N 15/29, C12N 15/82

(54) **METHOD FOR PRODUCING TRANSFORMED CELLS OF CORN OR CORN PLANT, NUCLEIC ACID CONSTRUCT, AND METHOD FOR INTRODUCING NUCLEIC ACID TO CELLS OR CORN PLANT**

(30) Priority: 28.12.2020 JP 2020218249; 21.12.2021 JP 2021206911
(71) Applicant: KANEKA CORPORATION, Osaka 530-8288 (JP)
(72) Inventor: ISHIDA, Yuji, Iwata-shi, Shizuoka 438-0802 (JP); ICHIKAWA, Masako, Iwata-shi, Shizuoka 438-0802 (JP)
(74) Representative: Hoffmann Eitle
(86) International application number: PCT/JP2021/048621
(87) International publication number: WO 2022/145427

(57) **Abstract**

The present invention relates to a method of producing a transformed cell or plant body of maize. The present invention also relates to a method of introducing a nucleic acid into a cell or plant body of maize. The present invention also relates to a nucleic acid construct. The method of producing a transformed cell or plant body of maize of the present invention includes overexpressing, in maize, 1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize, or 2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize.

## Description

### Technical Field

The present invention relates to a method of producing a transformed cell or plant body of maize. The present invention also relates to a method of introducing a nucleic acid into a cell or plant body of maize.

Moreover, the present invention relates to a nucleic acid construct. The nucleic acid construct of the present invention can be used for a method of producing a transformed cell or plant body of maize, or a method of introducing a nucleic acid into a cell or plant body of maize.

### Background Art

Maize is one of the three major crops in the world together with wheat and rice, and is a crop that is important as a source of starch or edible oil, and a raw material of bioethanol, as well as food or animal feed. Transformation of maize by electroporation was first reported in 1988 (Rhodes et al., 1988). Following this, various methods for transformation, such as a particle gun method (Fromm et al., 1990; Gordon-Kamm et al., 1990), a whisker method (Frame et al., 1994), an agrobacterium method (Ishida et al., 1996), and the like have been successively developed. Regarding a plant body material used for transformation of maize, various cells or tissues, such as protoplasts, suspension-cultured cells, calluses, growth point tissues, immature embryos, and the like, have been tested. Particularly, immature embryos have been frequently used for transformation by a particle gun method or an agrobacterium method because cells are actively divided, and calluses that have an ability to cause redifferentiation are easily formed. Moreover, media components, such as types or concentrations of plant body hormones, addition of minor components, such as copper, silver, etc., types of gelling agents, have been also improved.

Particularly when immature embryos of maize are cultured, two types of calluses having mutually different characteristics are obtained. One is a (heterogeneous) callus, which is called Type I callus and has a mild proliferation rate and an uneven shape. The other is a rapidly growing, compact, and friable callus, which is called Type II callus. The type of callus formed significantly varies depending on the variety of plant, and also varies depending on medium components. For example, it has been reported that the Type II calluses are actively formed in a medium where proline has been added to a N6 medium at a high concentration (Armstrong and Green, 1985).

As maize, moreover, "arduously culturable varieties," which are the varieties of plants that are difficult to culture compared to general varieties used for research (A188 etc.), such as B73, and elite varieties of maize derived from B73, have been known. It has been desired to develop a simple method of producing a transformed cell or plant body, particularly for varieties of maize that is considered "arduously culturable."

The WUSCHEL genes (WUS genes) are homeodomain transcription factors that improve or suppress expression of other genes. It has been known that most WUSCHEL genes (WUS genes) are asymmetrically expressed along an apical-base axis of an embryo. A plurality of genes having homeoboxes of WUS have been found in various plants, and such a gene pool is called WOX. It is considered that the WUS/WOX plays an important role in maintenance of stem cells at growth points, such as shoot apexes, root tips, and the like. The genes having the homeoboxes of WUS have been found in various plants, such as *Arabidopsis thaliana,* petunia, maize, rice, and the like.

As a novel method for maize transformation attempted in recent years, a method of overexpressing a morphogenetic gene in a maize cell has been known. As a method of efficiently obtaining a transformant of maize using a morphogenetic gene, a method of overexpressing two genes, ZmWUS2 gene and ZmBBM2 gene, has been known (Lowe et al., (2016, 2018)). However, it is generally difficult to obtain transformed plant bodies from cells obtained by strong constitutive expression of a morphogenetic gene. Even if transformed plant bodies are obtained, adverse effects, such as induction of abnormality of a phenotype and low fertility, are observed. These adverse effects may also appear in a method using a combination of two genes, ZmWUS2 gene and ZmBBM2 gene. To reduce the above-described adverse effects from the morphogenetic genes, for example, a method of removing unnecessary morphogenetic genes in a Cre-loxP system is adapted in a step prior to redifferentiation of the transformed cell with the genes in a plant. According to the above-described method, efficient production of transformed plant bodies of sorghum, rice, and sugarcane (Lowe et al., (2016)), as well as maize, has been realized. Moreover, Lowe et al., (2018) acquired a transformed plant body of maize within a short period of time according to a method of controlling expression of the genes with a tissue-specific promoter.

As described above, the maize transformation method using morphogenetic genes is an effective method, but the all methods known in the related art are methods of expressing two genes in combination. Therefore, a length of a vector used for transformation is long. As a result, it is difficult to construct a vector and difficulty of transformation also increases. Moreover, the effective method of removing ZmWUS2 genes and ZmBBM2 genes using the Cre-loxP system includes to allow the Cre-loxP system to function using the drought inducible promoter. Therefore, it is necessary to perform a drying process on transformation calluses, the complicated operations are necessary, and adverse effects, such as loss of reproduction ability in part of calluses due to damages from the drying process, may be observed.

The patent literature US 8,383,891 B2 discloses in Examples 6 and 7 that a plasmid DNA having a nucleic acid encoding a WUSCHEL polypeptide is introduced into maize immature embryos by a particle gun method, calluses are induced and redifferentiation culture is carried out on a N6 medium for 8 to 10 weeks to obtain transformants. However, Examples 6 and 7 of the above-mentioned latent literature are described in the format of the present tense, thus it is not certain whether transformants were actually obtained.

WO 2021/030242 A1 discloses that Sorghum WOX5 genes and Brachypodium WOX5 genes are expressed in monocotyledon plants using a promoter for strong constitutive expression of genes, such as a ubiquitin promoter (Pubi) and NOS promoter (Pnos) (Tables 2 and 3). However, the document does not clearly describe problems associated with use of these promoters, especially, abnormality of a phenotype of plants.

### Citation List

### [Patent Literature]

[PTL 1] WO 2018/224001 A1
[PTL 2] US 8,383,891 B2
[PTL 3] WO 2021/030242 A1
[PTL 4] WO 2014/157541 A1

### [Non-Patent Literature]

[NPL 1] Kyo et al. (2018) Coexpression of WUSCHEL related homeobox (WOX) 2 with WOX8 or WOX9 promotes regeneration from leaf segments and free cells in Nicotiana tabacum L. Plant Biotechnology 35; 23-30
[NPL 2] Ishida et al. (2007). Agrobacterium-mediated transformation of maize. Nat. Protoc. 2, 1614-1621. doi: 10.1038/nprot.2007.241
[NPL 3] Chiu et al. (1996) Engineered GFP as a vital reporter in plants. Current Biology 6(3); 325-330
[NPL 4] Todd J. Jones (2009) Maize Tissue Culture and Transformation: The First 20 Years. Molecular Genetic Approaches to Maize Improvement; 7-27
[NPL 5] Toda et al. (2019) An efficient DNA- and selectable-marker-free genome-editing system using zygotes in rice Nature plants 5; 363-368 https://doi.org/10.1038/s41477-019-0386-z
[NPL 6] Ohta et al. (1990) Construction and Expression in Tobacco of a β-Glucuronidase (GUS) Reporter Gene Containing an Intron Within the Coding Sequence Plant Cell Physiol. 31(6); 805-813
[NPL 7] Koiso et al. (2017) Development of gene expression system in egg cells and zygotes isolated from rice and maize. Plant Direct 1(3); e00010 https://doi.org/10.1002/pld3.10
[NPL 8] Lowe et al. (2018) Rapid genotype "independent" Zea mays L. (maize) transformation via direct somatic embryogenesis In Vitro Cellular & Developmental Biology - Plant 54; 240-252 https://doi.org/10.1007/s11627-018-9905-2
[NPL 9] Nardmann et al. (2007) WOX Gene Phylogeny in Poaceae: A Comparative Approach Addressing Leaf and Embryo Development. Molecular Biology and Evolution 24(11): 2474-84 https://doi.org/10.1093/molbev/msm182
[NPL 10] Armstrong and Green (1985) Establishment and maintenance of friable, embryogenic maize callus and the involvement of L-proline. Planta 164; 207-214
[NPL 11] Rhodes et al. (1988) Plant Regeneration from Protoplasts Isolated from Embryogenic Maize Cell Cultures. Nature Biotechnology 6; 56-60
[NPL 12] Fromm et al. (1990) Inheritance and Expression of Chimeric Genes in the Progeny of Transgenic Maize Plants. Biotechnology 8; 833-839
[NPL 13] Gordon-Kamm WJ et al. (1990) Transformation of Maize Cells and Regeneration of Fertile Transgenic Plants. Plant Cell 2; 603-618
[NPL 14] Frame et al. (1994) Production of fertile transgenic maize plants by silicon carbide whisker-mediated transformation. Plant Journal 6(6); 941-948
[NPL 15] Ishida et al. (1996) High efficiency transformation of maize (Zea mays L.) mediated by Agrobacterium tumefaciens. Nature Biotechnology 14; 745-750
[NPL 16] Lowe et al. (2016) Morphogenic Regulators Baby boom and Wuschel Improve Monocot Transformation. The Plant Cell 28; 1998-2015
[NPL 17] Prioli et al. (1989) Plant Regeneration and Recovery of Fertile Plants from Protoplasts of Maize (Zea Mays L.). Nature Biotechnology 7; 589-594
[NPL 18] Shillito et al. (1989) Regeneration of Fertile Plants from Protoplasts of Elite Inbread Maize. Nature Biotechnology 7; 581-587
[NPL 19] Yau, Y.Y., and Stewart, C.N., Jr. (2013). Less is more: strategies to remove marker genes from transgenic plants. BMC Biotechnol 13; 36
[NPL 20] Hiei et al. (2008). Agrobacterium-mediated transformation of rice using immature embryos or calli induced from mature seed. Nat. Protoc. 3, 824-834. doi: 10.1038/nprot.2008.46.

### Summary of Invention

### Technical Problem

There is a demand for a maize transformation method, which has a sufficient callus formation effect even with varieties that are difficult to culture, and can easily form a transformed plant body without complicated process, such as removal of morphogenic genes in the middle of culture. An object of the present invention is to provide a method of proliferating and culturing transformed cells from maize tissue samples while reducing abnormality of a phenotype of a transformed plant body of maize compared to that in the related art, and a method of producing a transformed plant body of maize. Preferably, the methods of the present invention are easier and more efficient than methods of the related art.

### Solution to Problem

The present invention includes, but is not limited to, the following aspects.

### [Aspect 1]

A method of producing a transformed cell or plant body of maize, the method including:
overexpressing, in maize,
   1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize; or
   2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize,
under the control of a promoter including a 35S promoter of Cauliflower mosaic virus.

### [Aspect 2]

The method according to Aspect 1, including:
overexpressing, in maize,
   1) the nucleic acid encoding a polypeptide including an amino acid sequence having at least 95% sequence identity with the amino acid sequence of SEQ ID NO: 2, the polypeptide having a function of promoting cell division of maize; or
   2) the nucleic acid encoding a polypeptide including an amino acid sequence having at least 95% sequence identity with the amino acid sequence of SEQ ID NO: 15, the polypeptide having a function of promoting cell division of maize,
under the control of the promoter including the 35S promoter of the Cauliflower mosaic virus.

### [Aspect 3]

The method according to Aspect 1 or 2,
wherein the overexpressing, in maize, the nucleic acid includes culturing immature embryos of the maize on a MS medium to undergo callus induction.

### [Aspect 4]

The method according to any one of Aspects 1 to 3, wherein the overexpressing, in maize, the nucleic acid includes culturing immature embryos of the maize for 2 weeks or longer and shorter than 8 weeks to undergo callus induction.

### [Aspect 5]

The method according to any one of Aspects 1 to 4, wherein the overexpressing, in maize, the nucleic acid includes culturing immature embryos of the maize on a medium including proline at a concentration higher than 6.1 mM to undergo callus induction.

### [Aspect 6]

The method according to Aspect 1 or 2,
wherein the overexpressing, in maize, the nucleic acid includes culturing immature embryos of the maize on a MS medium for 2 weeks or longer and shorter than 8 weeks to undergo callus induction.

### [Aspect 7]

The method according to any one of Aspects 1 to 6, wherein the promoter including the 35S promoter of the Cauliflower mosaic virus is
(i) a promoter having a sequence including the 35S promoter of the Cauliflower mosaic virus and a castor bean catalase intron placed downstream of the 35S promoter; or
(ii) a promoter composed of the 35S promoter of the Cauliflower mosaic virus.

### [Aspect 8]

The method according to any one of Aspects 1 to 7, wherein abnormality of a phenotype of the maize is reduced compared to a case where the nucleic acid is overexpressed in maize under the control of a promoter other than the promoter including the 35S promoter of the Cauliflower mosaic virus.

### [Aspect 9]

The method according to any one of Aspects 1 to 8, wherein the overexpressing, in maize, the nucleic acid is not carried out by expressing BABY BOOM.

### [Aspect 10]

A nucleic acid construct, including:
1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize, or
2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize; and
a promoter including a 35S promoter of Cauliflower mosaic virus for producing a nucleic acid in maize.

### [Aspect 11]

A method of introducing a nucleic acid into a cell or plant body of maize, the method comprising:
introducing the nucleic acid construct according to Aspect 10 and a desired nucleic acid into maize.

### [Aspect 12]

The method according to Aspect 11,
wherein a nucleic acid encoding BABY BOOM is not introduced into the maize.

### [Aspect 13]

A nucleic acid construct, including:
1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize, or
2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize;

a promoter including a 35S promoter of Cauliflower mosaic virus for producing a nucleic acid in maize; and
a desired nucleic acid.

### [Aspect 14]

The nucleic acid construct according to Aspect 13, wherein 1) the nucleic acid encoding an amino acid sequence of SEQ ID NO: 2, or the nucleic acid encoding a polypeptide including an amino acid sequence having at least 95% sequence identity with the amino acid sequence of SEQ ID NO: 2 and having the function of promoting cell division of maize, or
2) the nucleic acid encoding an amino acid sequence of SEQ ID NO: 15, or the nucleic acid encoding a polypeptide including an amino acid sequence having at least 95% sequence identity with the amino acid sequence of SEQ ID NO: 15 and having the function of promoting cell division of maize; and
the desired nucleic acid
   are directly bonded or bonded via a linker.

### [Aspect 15]

A method of introducing a nucleic acid into a cell or plant body of maize, the method including:
introducing the nucleic acid construct according to Aspect 13 or 14 into maize.

### Advantageous Effects of Invention

According to the present invention, a method of reducing abnormality of a phenotype of a transformed plant body of maize compared to a conventional maize transformation method where ectopic overexpression of morphogenetic genes is performed, and moreover, a method of easily and efficiently proliferating and culturing transformed cells from maize tissue samples, and a method of producing a transformed plant body of maize are provided.

### Description of Embodiments

The present invention includes, but is not limited to, the following embodiments. The technical and scientific terminology used in the present specification encompass the definitions generally understood by a person skilled in the art, unless otherwise stated in the present specification. The substances, materials, methods, and examples disclosed in the present specification are merely listed as examples, and are not intended to be used to limit a scope of the present invention. The phrase "in one embodiment" stated in the present specification means that the present invention is not limited to such an embodiment, and such an embodiment is nonrestrictive.

### 1. Method of producing transformed cell or plant body of maize

In one embodiment, the present invention relates to a method of producing a transformed cell or plant body of maize.

The method of the present invention includes, but is not limiting to,
overexpressing, in maize,
   1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize; or
   2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize,
under the control of a promoter including a 35S promoter of Cauliflower mosaic virus.

### (1) Nucleic acid

A nucleic acid used for the method of producing a transformed cell or plant body of maize is:
a nucleic acid 1) that is a nucleic acid encoding an amino acid sequence of SEQ ID NO: 2, or a nucleic acid encoding a polypeptide including an amino acid sequence having at least 95% sequence identity with the amino acid sequence of SEQ ID NO: 2,
the polypeptide having a function of promoting cell division of maize; or
a nucleic acid 2) that is a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize.

WUSCHEL-RELATED HOMEOBOX5 (WOX5) is a transcription factor that is considered to associate with cell division of plants, formation of lateral roots at an initial stage of embryonic development, and germination. The amino acid sequence of SEQ ID NO: 15 is an amino acid sequence encoded by a ZmWOX5b gene (ZmWOX5b cDNA (Transcript ID: DAA56787, Gramene ID: GRMZM2G116063_T01, SEQ ID NO: 14) that is one of maize variety B73-derived WOX5-related genes (ZmWOX). The amino acid sequence of SEQ ID NO: 15 is encoded by a nucleic acid sequence of SEQ ID NO: 14.

SEQ ID NO: 2 is an amino acid sequence encoded by a splice variant of ZmWOX5b cDNA (ZmWOX5bX1, Genbank accession No. XM_020550030, SEQ ID NO: 1). The amino acid sequence of SEQ ID NO: 2 is encoded by the nucleic acid sequence of SEQ ID NO: 1. SEQ ID NO: 15 is a sequence where amino acid residues 140-148 (bases 417-433 of the nucleic acid sequence of SEQ ID NO: 1) are deleted from the amino acid sequence of SEQ ID NO: 2.

The amino acids of SEQ ID NO: 15 are aligned in a manner that S (serine) of the 197th amino acid residue of SEQ ID NO: 2 (bases 588-591 agc of the nucleic acid of SEQ ID NO: 1) is substituted with I (isoleucine), G (glycine) of the 198th amino acid residue (bases 592-594 ggc of the nucleic acid sequence of Sequence Number 1) is substituted with A (alanine), and V (valine) of the 212th or 213th amino acid residue (or both are V) is deleted.

The base sequence of SEQ ID NO: 14 is a base sequence of SEQ ID NO: 1 where a of 231st base is substituted with c, a of the 588th base is substituted with c, g of the 590th base is substituted with t, g of the 593rd base is substituted with c, and gtc of the 634th to 636th bases or 637th to 639th bases are deleted.

The amino acid sequence encoded by the nucleic acid 1) or the nucleic acid 2) includes a variant of the amino acid sequence of SEQ ID NO: 2 or SEQ ID NO: 15, specifically includes an amino acid sequence having at least 85%, 86%, 87%, 88%, 89%, 90%, 91%, 92%, 93%, 94%, 95%, 96%, 97%, 98%, 99%, or 99.5% sequence identity with the amino acid sequence of SEQ ID NO: 2 or SEQ ID NO: 15. In one embodiment, an amino acid sequence encoded by the nucleic acid 1) or the nucleic acid 2) includes an amino acid sequence having at least 95% sequence identity with the amino acid sequence of SEQ ID NO: 2 or SEQ ID NO: 4.

In the present specification, the sequence identity % between two amino acid sequences can be determined by visual inspection and mathematical calculations. Moreover, the sequence identity % can be also determined using a computer program. Examples of such a computer program include BLAST, ClustalW, and the like. Particularly, various conditions (parameters) for the identity search using the BLAST program are described by Altschul et al. (Nucl. Acids. Res., 25, p. 3389-3402, 1997), and can be publicly acquired from a website of NCBI or DNA Data Bank of Japan (DDBJ) (BLAST Manual, Altschul et al., NCB/NLM/NIH Bethesda, MD 20894; Altschul et al.). Moreover, the sequence identity % can be also determined using a program, such as genetic information processing software GENETYX Ver. 7 (GENETYX), DNASIS Pro (Hitachi Software), Vector NTI (Infomax), and the like.

The nucleic acid 1) or the nucleic acid 2) may include a nucleic acid sequence of SEQ ID NO: 1, or a variant of a nucleic acid sequence of SEQ ID NO: 14.

Specifically, the nucleic acid 1) or the nucleic acid 2) may be, for example, an embodiment where "one, or two or more nucleotides are deleted, substituted, inserted, or added" in the nucleic acid sequence of SEQ ID NO: 1 or the nucleic acid sequence of SEQ ID NO: 14.

In the present specification, the phrase "one, or two or more nucleotides are deleted, substituted, inserted, or added" stated in association with a nucleic acid sequence means a nucleic acid sequence where one, or two or more nucleotides are deleted or substituted with other nucleotides, or other nucleotides are inserted, and/or other nucleotides are added in the subject nucleic acid sequence. The phrase "two or more nucleotides" means, but is not limited to, 600 nucleotides or less, 300 nucleotides or less, 150 nucleotides or less, 100 nucleotides or less, 50 nucleotides or less, 30 nucleotides or less, 20 nucleotides or less, 15 nucleotides or less, 12 nucleotides or less, 10 nucleotides or less, 8 nucleotides or less, 6 nucleotides or less, 5 nucleotides or less, 4 nucleotides or less, 3 nucleotides or less, or 2 nucleotides or less. Alternatively, two or more nucleotides means 15%, preferably 12%, 10%, 8%, 7%, 6%, 5%, 4%, 3%, 2%, 1%, or 0.5% of nucleotides relative to a total length of a nucleic acid sequence. Preferably, frameshift mutation of a sequence encoding amino acids is not caused by the deletion, substitution, insertion, or addition of nucleotide(s).

Alternatively, the nucleic acid 1) or the nucleic acid 2) includes or is composed of a nucleic acid sequence of SEQ ID NO: 1, or a nucleic acid sequence having at least 85%, 86%, 87%, 88%, 89%, 90%, 91%, 92%, 93%, 94%, 95%, 96%, 97%, 98%, 99%, or 99.5% sequent identity with the nucleic acid sequence of SEQ ID NO: 14. In one embodiment, the nucleic acid 1) or the nucleic acid 2) includes a nucleic acid sequence of SEQ ID NO: 1, or a nucleic acid sequence having at least 95% sequence identity with the nucleic acid sequence of SEQ ID NO: 14.

In the present specification, the identity % between two nucleic acid sequences can be determined by visual inspection and mathematical calculations. Moreover, the identity % can be also determined using a computer program. Examples of such a sequence comparison computer program include the BLASTN program, version 2.2.7, available from the website of National Library of Medicine, USA (https://blast.ncbi.nlm.nih.gov/Blast.cgi) (Altschul et al. (1990) J. Mol. Biol. 215: 403-10), the WU-BLAST2.0 algorism, and the like. As the setting of the standard default parameters for WU-BLAST2.0, the setting disclosed in the following internet site (http://blast.wustl.edu) can be used.

Alternatively, the nucleic acid 1) or nucleic acid 2) may be a nucleic acid that is hybridized with a nucleic acid sequence of the bases of SEQ ID NO: 1, or the nucleic acid sequence of SEQ ID NO: 14 under stringent conditions. Alternatively, the nucleic acid 1) or nucleic acid 2) may be a nucleic acid that is hybridized with a sequence complementary to the nucleic acid sequence of SEQ ID NO: 1 or the nucleic acid sequence of SEQ ID NO: 3 under stringent conditions.

In the present specification, the phrase "under stringent conditions" means that hybridization is carried out under a moderately or highly stringent conditions. Specifically, the moderately stringent conditions can be easily determined by a person of ordinary skill in the art, for example, based on a length of a DNA. Basic conditions are disclosed in Sambrook et al., Molecular Cloning: A Laboratory Manual, Vol. 3, Chpt. 6-7, Cold Spring Harbor Laboratory Press, 2001. Preferably, the moderately stringent conditions are, as hybridization conditions, conditions of 1×SSC to 6×SSC and 42°C to 55°C, more preferably conditions of 1×SSC to 3×SSC and 45°C to 50°C, and the most preferably 2×SSC and 50°C. When a hybridization solution includes approximately 50% of formamide, for example, a temperature that is lower than the above-mentioned temperature by 5°C to 15°C is selected. As the washing conditions, conditions of 0.5×SSC to 6×SSC and 40°C to 60°C are exemplified. At the time of hybridization and washing, generally, 0.05% to 0.2%, preferably approximately 0.1% of SDS may be added. The highly stringent conditions can be also easily determined by a person skilled in the art, for example, depending on a length of DNA. Generally, the highly stringent conditions include hybridization and/or washing at the higher temperature and/or lower salt concentration than the moderately stringent conditions. The hybridization conditions are, for example, conditions of 0.1×SSC to 2×SSC and 55°C to 65°C, more preferably conditions of 0.1×SSC to 1×SSC and 60°C to 65°C, and the most preferably conditions of 0.2×SSC and 63°C. As the washing conditions, conditions of 0.2×SSC to 2×SSC and 50°C to 68°C, more preferably conditions of 0.2×SSC and 60°C to 65°C are exemplified.

In one embodiment, a nucleic acid used for the method of producing a transformed cell or plant body is:
1) a nucleic acid encoding a polypeptide including an amino acid sequence having at least 95% sequence identity with an amino acid sequence of SEQ ID NO: 2, the polypeptide having a function of promoting cell division of maize; or
2) a nucleic acid encoding a polypeptide including an amino acid sequence having at least 95% sequence identity with an amino acid sequence of SEQ ID NO: 15, the polypeptide having a function of promoting cell division of maize.

The nucleic acid 1) and the nucleic acid 2) each encode a polypeptide that mediates cell division of maize. The nucleic acid 1) and the nucleic acid 2) are WOX5-related genes. In one embodiment, the nucleic acid 1) and the nucleic acid 2) maintain functions the WOX5-related genes have (including functions of a protein encoded by WOX5-related genes). One example of functions encoded by the WOX5-related gene includes cell division of plants (including induction of rooting), maintaining of stem cells, etc. In one embodiment, the nucleic acid 1) and nucleic acid 2) each encode a polypeptide having a function of promoting cell division of maize. The polypeptide "having a function of promoting cell division" means, for example, that, when the polypeptide is ectopically overexpressed in a plant cell, the cell is more actively divided than generally.

### (2) Plant

A plant that is a target of the method of producing a transformed cell or plant body is maize.

For production of transformed cells or plant bodies of maize, compact and friable calluses, which actively proliferate and are called Type II calluses, are generally used, when maize suspension-cultured cells are produced (Prioli, 1989). Moreover, Shillito et al. (1989) also mentioned a non-mucilaginous characteristic regarding calluses used for suspension cultures. On the other hand, (heterogeneous) calluses having a mild proliferation rate and uneven shapes, which are called Type I calluses, are considered to be unsuitable for production of suspension-cultured cells.

In one embodiment, maize that is a target of the method of producing a transformed cell or plant body may be maize of "arduously culturable varieties." The maize of "arduously culturable varieties" includes varieties that are difficult to culture compared to varieties (A188 etc.) generally used for research, and examples of the arduously culturable varieties include B73 and elite varieties of maize derived from B73.

### (3) Overexpression

In the present invention, the method includes overexpressing the nucleic acid in a plant under the control of a promoter including a 35S promoter of Cauliflower mosaic virus.

The term "overexpressing" (may be merely referred to as "expressing" in the present specification) means artificially expressing a nucleic acid that is not expressed in the natural state, or artificially expressing the larger amount of nucleic acid than the amount expressed in the natural state.

A method of overexpressing a nucleic acid is not particularly limited. Any of methods known in the related art may be used, provided that the method is a method capable of overexpressing a nucleic acid in a plant (including any embodiments of eggs, pollens, seeds, cells, immature embryos, mature embryos, calluses, matured bodies, and the like). Although it is not particularly limited, a gene may be introduced into a plant by a gene transfer method where a foreign gene is incorporated into a vector etc., and an agrobacterium method is carried out. Moreover, a gene present in a plant may be overexpressed by genome editing, etc.

A gene transfer method of introducing a foreign nucleic acid is not particularly limited, and includes methods known in the related art, such as an agrobacterium method, polyethylene glycol (PEG)-mediated transformation, a whisker method, microinoculation, glass bead transformation, a particle gun method, electroporation, and the like.

The genome editing method to overexpress a gene present in a plant is not particularly limited. Examples of the genome editing method include CRISPR-Cas, TALLEN, zinc finger-mediated mutagenesis, targeting induced local lesions IN genomes (TILLING), homologous recombination, oligonucleotide-directed mutagenesis, meganuclease-mediated mutagenesis, and any combination of the foregoing methods. Examples of the method include, but are not limited to, a method where a promoter of an endogenous gene is edited and overexpressed, and a method where part of an endogenous gene is edited to be the gene sequence of the present invention.

In one embodiment, the overexpression includes an embodiment of transient or site-specific overexpression of a nucleic acid, as well as the embodiment of constitutive overexpression of the nucleic acid.

The overexpressing the nucleic acid in maize includes, but is not limited to, the following embodiments:
(i) the overexpressing, in maize, the nucleic acid includes culturing immature embryos of the maize on a MS medium to undergo callus induction;
(ii) the overexpressing, in maize, the nucleic acid includes culturing immature embryos of the maize for 2 weeks or longer and shorter than 8 weeks to undergo callus induction; and/or,
(iii) the overexpressing, in maize, the nucleic acid includes culturing immature embryos of the maize on a medium including proline at a concentration higher than 6.1 mM to undergo callus induction.

The overexpressing, in maize, the nucleic acid may include, but is not limited to, any one selected from (i) to (iii), or a combination of any two or more selected from (i) to (iii). In one embodiment, for example, the overexpressing, in maize, the nucleic acid including a combination of (i) and (ii) may include culturing immature embryos of the maize on a MS medium for 2 weeks or longer and shorter than 8 weeks to undergo callus induction.

The callus induction medium is not particularly limited, and any known medium, such as a N6 medium (a medium composed of N6 basal inorganic salts (N6 major salts, N6 minor salts, Fe)), a MS medium (a medium composed of MS basal inorganic salts (MS major salts, MS minor salts, Fe)), and the like may be used. Compositions of callus induction media known in the related art are presented in Table 1 below.

### Table 1 Compositions of inorganic salts etc. of media used for culturing various plants

**[Table 1]**

| mg/L Medium | | N6 | B5 | R2 | CC | AA(T&H) | MS | LS |
|---|---|---|---|---|---|---|---|---|
| Major salts | (NH₄)₂SO₄ | 463 | 134 | 330 | | | | |
| | MgSO₄•7H₂O | 185 | 500 | 246 | 247 | 250 | 370 | 370 |
| | KCl | | | | | 2950 | | |
| | CaCl₂•2H₂O | 166 | 150 | 147 | 588 | 150 | 440 | 440 |
| | KNO₃ | 2830 | 2528 | 4044 | 1212 | | 1900 | 1900 |
| | NH₄NO₃ | | | | 640 | | 1050 | 1650 |
| | NaH₂PO₄•H₂O | | 150 | | | 150 | | |
| | NaH₂PO₄•2H₂O | | | 312 | | | | |
| | KH₂PO₄ | 400 | | | 136 | | 170 | 170 |
| Minor salts | MnSO₄•4H₂O | 4.4 | 13.2 | 20.3 | **11.15** | 13.2 | 22.3 | 22.3 |
| | ZnSO₄•7H₂O | 1.5 | 2 | 2.2 | 5.76 | 2 | 8.6 | 8.6 |
| | CuSO₄•5H₂O | | 0.025 | 0.196 | 0.025 | 0.025 | 0.025 | 0.025 |
| | CoCl₂•6H₂O | | 0.025 | 0.025 | | 0.025 | 0.025 | 0.025 |
| | CoSO₄•H₂O | | | | 0.028 | | | |
| | KI | 0.8 | 0.75 | | 0.83 | 0.75 | 0.83 | 0.83 |
| | H₃BO₃ | 1.6 | 3 | 2.86 | 3.1 | 3 | 6.2 | 6.2 |
| | NaMoO₄•2H₂O | | 0.25 | 0.125 | 0.24 | 0.25 | 0.25 | 0.25 |
| Fe | FeSO₄•7H₂O | 27.85 | 27.85 | 27.85 | 27.85 | 27.85 | 27.85 | 27.85 |
| | Na₂EDTA | 37.25 | 37.25 | 37.25 | 37.25 | 37.25 | 37.25 | 37.25 |

In one embodiment, the MS medium may be a medium having the concentrations of the MS basal inorganic salts (all the major salts, minor salts, and Fe) of Table 1 as they are, or a diluted or concentrated medium of the MS medium presented in Table 1. In case of a diluted medium, the concentrations of the MS basal inorganic salts are preferably ×0.9, ×0.8, ×0.7, ×0.6, or ×0.5. In case of a concentrated medium, the concentrations of the MS basal inorganic salt are preferably ×1.1, ×1.2, ×1.3, ×1.4, ×1.5, ×1.6, ×1.7, ×1.8, ×1.9, or ×2.0.

In one embodiment, the MS medium may be a medium composed of MS basal inorganic salts (all major salts, minor salts, and Fe), or a mixture medium with other basal inorganic salts. In case of the mixture medium, a ratio of the MS basal inorganic salts to other basal inorganic salts is preferably 9:1, 8:2, 7:3, 6:4, or 5:5.

In one embodiment, the minor salts may include inorganic salts other than the minor salts of the MS medium. In this case, however, the major salts preferably constitute a MS medium itself that is not mixed with other minor salts, or preferably constitute a medium that is diluted or concentrated.

Preferably, immature embryos of maize are cultured on a MS medium to undergo callus induction.

Preferably, immature embryos of maize are cultured for shorter than 8 weeks, 7 weeks or shorter, 6 weeks or shorter, 5 weeks or shorter, or 4 weeks or shorter to undergo callus induction. Preferably, immature embryos of maize are cultured for 1 week or longer, 2 weeks or longer, or 3 weeks or longer to undergo callus induction. In one embodiment, immature embryos of maize are cultured on a MS medium for 1 week or longer and shorter than 8 weeks, 2 weeks or longer and shorter than 8 weeks, 3 weeks or longer and shorter than 8 weeks, 1 week or longer and 7 weeks or shorter, 2 weeks and longer and shorter than 7 weeks, 3 weeks and longer and 7 weeks or shorter, 1 week or longer and 6 weeks or shorter, 2 weeks and longer and shorter than 6 weeks, 3 weeks or longer and 6 weeks or shorter, 1 week or longer and 5 weeks or shorter, 2 weeks or longer and shorter than 5 weeks, or 3 weeks or longer and 5 weeks or shorter to undergo callus induction.

In one embodiment, immature embryos of maize are cultured on a medium including proline at a high concentration, for example, a medium including proline at a concentration higher than 6.1 mM, to undergo callus induction. The proline concentration of the callus induction medium is, but is not limited to, 6.1 mM or higher, 7 mM or higher, 10 mM or higher, 15 mM, 18 mM or higher, 20 mM or higher, or 25 mM or higher.

In one embodiment, the method of producing a transformed cell or plant body of maize includes overexpressing the nucleic acid in maize under the control of a promoter for producing a nucleic acid.

In one embodiment, a promoter, which is relatively weak and whose expression level tends to start decreasing around at the point of or after redifferentiation culture (e.g., a 35S promoter (P35S) of Cauliflower mosaic virus) is more preferred to a promoter (e.g., a ubiquitin promoter) that is generally considered to generate strong expression, because the result has been already obtained such that use of the promoter of low expression contributes to reduction in abnormality of a phenotype of a plant.

In one embodiment, the method of producing a transformed cell or plant body of maize includes overexpressing, in maize, the nucleic acid under the control of a promoter including a 35S promoter (P35S) of Cauliflower mosaic virus. Example 16 of the present specification indicates that P35S is relatively weak and tends to start decreasing the expression rate around at the point of or after redifferentiation culture, compared to a ubiquitin promoter that is generally considered to generate strong expression.

The "promoter including a 35S promoter of Cauliflower mosaic virus" may include one, or two or more additional gene expression regulatory sequences, provided that the promoter is a promoter "including" the 35S promoter of the Cauliflower mosaic virus. Examples of the additionally included gene expression regulatory sequence include, but are not limited to, intron sequences, 5' untranslated regions, enhancer sequences, and the like. Examples of the intron sequences include castor bean catalase intron sequences, and various alcohol dehydrogenase intron sequences, and the like. Examples of the 5' untranslated regions include 5' untranslated regions of various actin genes, various ubiquitin genes, various RuBisCO genes, various tubulin genes, tobacco etch virus (TEV), tobacco mosaic virus (TMV), Kyuri green mottle mosaic virus (KGMMV), and the like. Examples of the enhancer sequences include Hepta-repeats of maize, P1-rr distal enhancers, Distal cis-elements, tb1 enhancers, Block C, Region C, and L3 enhancers of Arabidopsis thaliana, AB80 enhancers of peas, enhancer sequences of Rous sarcoma virus RSV of retroviruses, cytomegalovirus (CMV), Cauliflower mosaic virus (CaMV), alfalfa mosaic virus (AMV), figwort mosaic virus, and the like.

Moreover, two or more promoters, such as a 35S promoter of Cauliflower mosaic virus and other promoters may be linked together.

An additionally included gene expression regulatory sequence may be placed downstream or upstream of the 35S promoter of Cauliflower mosaic virus. In one embodiment, an additionally included promoter is placed downstream of the 35S promoter of Cauliflower mosaic virus.

The promoter including the 35S promoter of Cauliflower mosaic virus is, but is not limited to:
(i) a promoter having a sequence including the 35S promoter of the Cauliflower mosaic virus and a castor bean catalase intron placed downstream of the 35S promoter; or
(ii) a promoter composed of the 35S promoter of the Cauliflower mosaic virus.

In one embodiment, the overexpressing the nucleic acid in maize is not carried out by expressing a BABY BOOM (BBM) gene. BBM is an embryogenesis-related gene. As one of BBM genes of maize, for example, ZmBBM2 has been known (Genbank accession No. XM008676474, SEQ ID NO: 9).

### (4) Effect of method of producing transformed cell or plant body of maize

According to the method of producing a transformed cell or plant body of maize, abnormality of a phenotype of maize is preferably reduced compared to a case where a nucleic acid is overexpressed in maize under the control of a promoter other than the promoter including the 35S promoter of the Cauliflower mosaic virus.

In the present specification, the term "abnormality of a phenotype" means that maize has some kind of morphological abnormality (mainly in gross morphology), and the kind of abnormality is not particularly limited. In one embodiment, the "abnormality of a phenotype" includes the abnormality of the phenotype where expressions of a maize plant differ from regular expressions, such as including at least one of the following characteristics: stocks do not grow straight; extreme dwarfing is observed; shrinkages of leaves are observed; tips of roots are enlarged; rooting is delayed; a tassel and/or ears are not differentiated; and the like. Alternatively, the "abnormality of a phenotype" includes albinism and the like.

Compared to the case where the nucleic acid is overexpressed in maize under the control of a promoter other than the promoter including the 35S promoter of the Cauliflower mosaic virus, preferably, the abnormality of the phenotype of the maize is reduced by 40% or greater, 50% or greater, 60% or greater, 70% or greater, 80% or greater, 90% or greater, 95% or greater, or 98% or greater. When transformation is performed by the method of producing a transformed cell or plant body of maize, preferably, an occurrence rate of abnormality of the phenotype among whole cells or plant bodies transformed is 60% or less, 50% or less, 40% or less, 40% or less, 30% or less, 20% or less, 10% or less, 5% or less, 3% or less, or 1% or less. When transformation is performed by the method of producing a transformed cell or plant body of maize, more preferably, abnormality of the phenotype does not occur.

The observed plants in Examples 12 and 13 of the present specification included: plants where stocks did not grow straight (and/or shrinkage of leaves was observed) and reproductive tissues were not differentiated (resulting in no fertility); plants where stocks did not grow straight (and/or shrinkage of leaves was observed) but reproductive tissues were differentiated and seeded; plants where stocks grew straight and shrinkage of leaves did not observed, but reproductive tissues were not differentiated (resulting in no fertility); and plants where cross-fertilization was carried out but resulting in no fertility. In Examples, the number of plants that ultimately seeded (were fertilized) was counted as the fertilized products. As a result, the fertility rate was significantly improved when transformation was performed by the method of producing a transformed cell or plant of maize.

In one embodiment, the method of producing transformed cell or plant body of maize improves a fertility rate compared to a case where a nucleic acid is overexpressed in maize under the control of a promoter other than the promoter including a 35S promoter of Cauliflower mosaic virus. The fertility rate may be influenced by external factors, such as cultivation environments and the like, but generally speaking, the lighter the degree of abnormality of the phenotype is, the higher the fertility rate is. If the abnormality of the phenotype is severe, differentiation of reproductive tissues is inhibited, resulting in a low fertility rate. Namely, the fertility rate reflects the degree of abnormality of the phenotype. An effect "abnormality of the phenotype of maize is reduced" includes significant improvement of a fertility rate as described above.

In one embodiment, when transformation is performed by the method of producing a transformed cell or plant body of maize, the fertility rate preferably improves by 20% or greater, 40% or greater, 45% or greater, 50% or greater, 60% or greater, 80% or greater, or 100% or greater, compared to the case where a nucleic acid is overexpressed in maize under the control of a promoter other than the promoter including a 35S promoter of Cauliflower mosaic virus. In one embodiment, when transformation is performed by the method of producing a transformed cell or plant body of maize, the fertility rate is preferably 48% or greater, 50% or greater, 55% or greater, or 60% or greater.

According to the method of producing a transformed cell or plant body of maize, preferably, effects of improving at least one selected from the group consisting of (i) a callus formation rate, (ii) a redifferentiation efficiency rate of transformed shoots, and (iii) a production efficiency rate of transformed rooting plants, compared to the case where the nucleic acid is not overexpressed in maize, are obtained.

The "callus formation rate" is a ratio of the immature embryos with which calluses are formed relative to all of the cultured immature embryos of maize (the number of immature embryos with which calluses are formed/the number of immature embryos that are cultured). The formation of a callus can be confirmed, for example, by observing an enlarged cell from the scutellum of the immature embryo sample under a microscope.

The "redifferentiation efficiency rate of transformed shoots" is a ratio of the number of the immature embryos with which the transformed shoots are re-differentiated relative to the number of the immature embryos of maize with which calluses are formed, or the number of the immature embryos of maize to which transfer of a nucleic acid and culturing are performed (the number of immature embryos with which transformed shoots are re-differentiated/the number of immature embryos with which calluses are formed or the number of immature embryos to which transfer of a nucleic acid and culturing are performed). The "redifferentiation of transformed shoots" can be confirmed, for example, by performing a redifferentiation culture under selective conditions by which untransformed calluses are not re-differentiated, and observing the presence of actively re-differentiated shoots.

The "production efficiency rate of transformed rooting plants" is a ratio of the number of plants with which shooting and rooting are confirmed and transformation is confirmed relative to the number of immature embryos to which transfer of a nucleic acid and culturing are performed, or the number of immature embryos with which calluses are formed (the number of immature embryos with which products where shooting and rooting are confirmed and transformation is confirmed are produced/the number of immature embryos where a nucleic acid is introduced or cultured, or the number of immature embryos with which calluses are formed). The "rooting" can be confirmed, for example, by observing the presence of a plurality of re-differentiated roots having shoots in a rooting medium. The "transformation" can be performed, for example, by any method known in the related art, such as a method where a marker gene is included in a nucleic acid to be introduced, and expression of the marker gene is confirmed, and the like. For example, a product where a drug resistance gene, which is a positive marker, is introduced and transformed can be selected by a selectable drug (e.g., phosphinothricin (PPT)/glufosinate, hygromycin, kanamycin, and the like) corresponding to the introduced drug resistance gene. Moreover, a fluorescent marker gene (e.g., a DsRed gene, a GFP gene, and the like), which is a visible marker, is introduced together, cells with which the fluorescent emission is observed are cultured, and the transformed cells or plant bodies can be acquired.

The "improving" means, but is not limited to, improving (i) a callus formation rate, (ii) a redifferentiation efficiency rate of transformed shoots, or (iii) a production efficiency rate of transformed rooting plants, for example, by 1.2 times or greater, 1.5 times or greater, 2 times or greater, 2.5 times or greater, 3 times or greater, 4 times or greater, or 5 times or greater, compared to the case where the nucleic acid is not overexpressed. Alternatively, the "improving" means that formation of plant calluses, redifferentiation of a plant, or gene transfer, which is not possible when the nucleic acid is not overexpressed, has become possible.

At least one, preferably two, and more preferably all of (i) a callus formation rate, (ii) a redifferentiation efficiency rate of transformed shoots, and (iii) a production efficiency rate of transformed rooting plants is improved, compared to the case where the nucleic acid is not overexpressed in maize.

### 2. Nucleic acid construct (Aspect A)

In one embodiment, the present invention relates to a nucleic acid construct.

The nucleic acid construct includes, but is not limited to:
1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize, or
2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize; and
a promoter for producing a nucleic acid in maize.

The "nucleic acid" of 1) or 2) and the "promoter" are as described in "1. Method of producing transformed cell or plant body of maize" above.

The nucleic acid of 1) or 2) and the promoter for expressing the nucleic acid in a plant body may be linked with an arbitrary vector. The "vector" is a nucleic acid molecule for amplifying, maintaining, and introducing a recombined nucleic acid used in the gene recombination technology. An entire part of a product where a nucleic acid and a promoter for expressing the nucleic acid in a plant are linked with a vector may be referred to as a nucleic acid construct or vector. The vector may have a straight-chain structure or a cyclic structure, preferably a cyclic structure.

The nucleic acid and the promoter for expressing the nucleic acid in a plant can be linked with a readily available recombination vector by a commonly used method. The vector is not particularly limited, provided that the vector is used to achieve an intended effect in plant cells. For examples, vectors, such as pBI vectors, pBluescript vectors, pUC vectors, and the like, may be used. Examples of the pBI vectors include pBI121, pBI101, pBI101.2, pBI101.3, pBI221, and the like. A binary vector, such as a pBI vector, is preferred because a desired nucleic acid can be introduced into a plant via Agrobacterium. Moreover, examples of the pBluescript vectors include pBluescript SK(+), pBluescript SK(-), pBluescript II KS(+), pBluescript II KS(-), pBluescript II SK(+), pBluescript II SK(-), and the like. Examples of the pUC vectors include pUC19, pUC119, and the like. The pBluescript vector and pUC vector are preferred because a nucleic acid can be directly introduced into a plant. In addition, binary vectors, such as pGreen series (www.pgreen.ac.uk), pCAMBIA series (www.cambia.org), pLC series (WO2007/148819), and the like, or super binary vectors, such as pSB11 (Komari et al, 1996, Plant J, 10: 165-174), pSB200 (Komori et al, 2004, Plant J, 37: 315-325), and the like may be preferably used. Moreover, a vector prepared by combining parts of the foregoing vectors may be also preferably used.

The vector may further include a transformant identification marker. As the marker, for example, a drug-selectable marker gene may be used. As the drug-selectable marker gene, any gene known in the related art may be used, and the drug-selectable marker gene is not particularly limited. Specific examples of the drug-selectable marker gene include various drug-selectable marker genes, such as gentamicin resistance genes, kanamycin resistance genes, ampicillin resistance genes, spectinomycin resistance genes, tetracycline resistance genes, and hygromycin resistance genes. Moreover, phosphinothricin acetyltransferase genes (bar) having resistance to herbicide phosphinothricin (PPT), or the like may be used. Moreover, fluorescent marker genes, such as DsRed2 (TaKaRa-Bio) and GFP, may be used.

The vector may further include a transcription terminator and the like. The transcription terminator is not particularly limited, provided that the transcription terminator is a section of a sequence functioning as a transcription terminator, and may be selected from transcription terminators known in the related art. The transcription terminator may be selected depending on a promoter used. For example, a transcriptional termination region of Cauliflower mosaic virus 35S (CaMV35S terminator), a transcriptional termination region of a nopaline synthase gene (Nos terminator), or the like can be used. After the recombinant expression vector is introduced into a plant cell, a phenomenon where a transcript that is longer than necessary is synthesized and the like can be prevented by arranging a transcription terminator in an appropriate site.

Moreover, the recombinant expression vector may include another nucleic acid segment. Such a nucleic acid segment is not particularly limited, but examples of the additional nucleic acid segment include a transformant selectable marker, an enhancer, a base sequence for increasing translation efficiency, and the like. Moreover, the recombinant expression vector may further include a T-DNA region. The T-DNA region can enhance the efficiency of gene transfer especially when the recombinant expression vector is introduced into a plant using Agrobacterium. Note that, the number of T-DNAs per recombinant expression vector is not limited, and may be appropriately selected according to the intended purpose.

Examples of the nucleotide sequence for enhancing translation efficiency include a tobacco mosaic virus-derived omega sequence. The translation efficiency of the gene can be enhanced by placing the omega sequence in an untranslated region (5'UTR) of a promoter.

Moreover, examples of the enhancer include an enhancer region including the sequence of the upstream side within a CaMV35S promoter, introns, 5'-UTR, 3'-UTR, and the like. For example, a castor bean catalase intron, which is used in Example 10 of the present specification, functions as an enhancer for the upstream Cauliflower mosaic virus 35S promoter. As described above, the recombinant expression vector may include various nucleic acid segments according to the intended purpose.

The nucleic acid construct (Aspect A) of the present invention can be used for the method of producing a transformed cell or plant body of maize and/or the method of introducing a nucleic acid into a cell or plant body of maize of the present invention. The present invention includes use of the nucleic acid construct in the method of producing a transformed cell or plant body of maize and/or the method of introducing a nucleic acid into a cell or plant body of maize. The present invention includes the nucleic acid construct used in the method of producing a transformed cell or plant body of maize and/or the method of introducing a nucleic acid into a cell or plant body of maize.

### 3. Method of introducing nucleic acid into cell or plant body of maize (Aspect A)

In one embodiment, the present invention relates to a method of introducing a nucleic acid into a cell or plant body of maize (Aspect A).

The method of introducing a nucleic acid into a cell or plant body of maize (Aspect A) includes introducing the nucleic acid construct of the present invention and a desired nucleic acid into maize.

The method of introducing the nucleic acid construct of the present invention and a desired nucleic acid into maize is not particularly limited, and any method known in the related art can be used. For example, any known method in the related art described as the gene transfer method to introduce a foreign nucleic acid in "1. Method of producing transformed cell or plant body of maize," such as an agrobacterium method, polyethylene glycol (PEG)-mediated transformation, a whisker method, microinoculation, glass bead transformation, a particle gun method, electroporation, and the like, can be used.

The "cell" of maize includes any cell of maize, such as egg cells, sperm cells, fertilized egg cells, fertilized egg cells starting division, cells constituting tissues, and a cell mass obtained by culturing any of the foregoing cells (e.g., calluses). The "plant body" of maize includes a matured body, parts (e.g., leaves, stems, roots, flowers, etc.) of the body, or the like of maize.

The "desired nucleic acid" (Gene of interest: GOI) is a nucleic acid having an arbitrary sequence to be introduced into a plant (maize) cell, and is not particularly limited. The nucleic acid may be of a nucleic acid sequence encoding an amino acid sequence (a structural gene), or a nonstructural gene. A desired promoter, terminator, or enhancer may be linked with the target nucleic acid sequence. For example, the sequence may be a set of a gene and a promoter that can create a desired morphology (phenotype) through overexpression within a plant, or a nucleic acid sequence that can create a desired morphology through suppression of an endogenous gene within a plant, such as RNAi. Moreover, the sequence may be a nucleic acid sequence for genome editing, such as a gene or guide sequence to encode Cas protein.

The length of the nucleic acid is not particularly limited, but the length of the nucleic acid is preferably a suitable length to be introduced into a plant cell together with the nucleic acid construct (Aspect A) and expressed in the plant cell. The length is, but is not limited to, 100 kbp or less, preferably 50 kbp or less, and more preferably 30 kbp, 10 kbp or less, or 1 kbp or less.

A method of introducing the nucleic acid construct (Aspect A) and a desired nucleic acid into a plant is not particularly limited. The method may be a method of splicing a foreign gene into a vector or the like to introduce the gene into a plant by a gene transfer method, such as an agrobacterium method. For example, any method known in the related art described as the gene transfer method to introduce a foreign nucleic acid in "1. Method of producing transformed cell or plant body of maize," such as an agrobacterium method, polyethylene glycol (PEG)-mediated transformation, a whisker method, microinoculation, glass bead transformation, a particle gun method, electroporation, and the like, can be used. Alternatively, an embodiment where a gene present within a plant is edited and expressed by a method, such as genome editing and the like, may be also included as the method of introducing the nucleic acid construct (Aspect A) and the desired nucleic acid into a plant.

The order of the introduction is not restricted, as long as both nucleic acids, namely, the nucleic acid construct (Aspect A) and the desired nucleic acid, are introduced at some point during a step of introducing the nucleic acid into a cell or plant body of maize. The nucleic acid construct and the desired nucleic acid may be introduced at the same time (co-transformation). Alternatively, the nucleic acid construct is introduced first to produce a transformed cell or plant, followed by introducing the desired nucleic acid into the transformed cell or plant, or vice versa.

The nucleic acid construct and the nucleic acid may be each present in separate vectors, or may be both present in the same vector. When both the nucleic acid construct and the nucleic acid are present in one vector, both the nucleic acid construct and the nucleic acid may be under the control of the same promoter or under the control of different promoters. Moreover, the nucleic acid construct and the desired nucleic acid may be introduced as straight-chain fragments, followed by expression.

The co-transformation is transformation where 2 or more independent exogenous genetic materials are transformed simultaneously. Examples of the co-transformation method include: a method of introducing a plurality of binary vectors each including a plurality of T-DNAs for one gene transfer (multi-vector method); a method of introducing one binary vector having two or more T-DNAs for one gene transfer (1 vector/multiple T-DNA method); a method of introducing one binary vector for one gene transfer, where the vector has only one T-DNA, and the one T-DNA composed of 2 or more genes using two or more right border sequences is placed (1 vector/2 border method: Yau and Stewart, 2013); a method of introducing one binary vector for one gene transfer, where the vector has only one T-DNA, and the T-DNA includes two or more promoters, desired nucleic acids, or terminators (1 vector/1 T-DNA method); and the like. Any of the above-listed methods can be carried out in the present invention.

A type of the vector for introducing the nucleic acid construct and a desired nucleic acid into a plant is not particularly limited. An arbitrary vector as described in "2. Nucleic acid construct (Aspect A)" may be used. In the case of the multi-vector method, an arbitrary combination of the foregoing vectors may be used.

Moreover, the nucleic acid construct and/or desired nucleic acid may undergo transient expression or stable expression. In one embodiment, without limiting, the nucleic acid construct or the desired nucleic acid undergoes transient expression.

In one embodiment, the method of introducing a nucleic acid in a cell or plant body of maize does not include introducing a nucleic acid encoding the BABY BOOM into maize.

### 4. Nucleic acid construct (Aspect B)

In one embodiment, the present invention relates to a nucleic acid construct (Aspect B).

The nucleic acid construct(Aspect B) includes, but is not limited to:
1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize, or
2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize;

a promoter for producing a nucleic acid in maize; and
a desired nucleic acid.

The "nucleic acid" of 1) or 2), and the "promoter" are as described above in "1. Method of producing transformed cell or plant body of maize." The "desired nucleic acid" is as described above in "3. Method of introducing nucleic acid in cell or plant body of maize."

The nucleic acid of 1) or 2) and the promoter for expressing the nucleic acid in a plant may be bonded to an arbitrary vector. The "vector" is as described above in "2. Nucleic acid construct (Aspect A)."

The nucleic acid construct (Aspect B) is expressed in a plant to obtain a fusion protein between a protein encoded by the nucleic acid of 1) or 2) and a protein encoded by a desired nucleic acid, i.e., a chimeric protein. A method of preparing a chimeric protein is not particularly limited. For example, the nucleic acid construct may be expressed by any gene engineering method known in the related art.

In one embodiment, without limiting, the nucleic acid of 1) or 2) and the desired nucleic acid to be expressed in a plant may be directly bonded or bonded via a linker. In one embodiment, the desired nucleic acid to be expressed in a plant is bonded to the 3' side of the nucleic acid of 1) or 2).

In a chimeric protein obtained by expressing the nucleic acid construct (Aspect B) in a plant, the protein encoded by the nucleic acid of 1) or 2) and the protein encoded by the desired nucleic acid may be directly bonded, or bonded via a linker. The linker is not particularly limited, but examples of the linker include amino acid linkers. In one embodiment, a length of an amino acid linker is, but is not limited to, 1 amino acid residue or more, 2 amino acid residues or more, 3 amino acid residues or more, 4 amino acid residues or more, 5 amino acid residues or more, 8 amino acid residues or more, 10 amino acid residues or more, or 12 amino acid residue or more. In one embodiment, the length of the amino acid linker is, but is not limited to, 50 amino acid residues or less, 40 amino acid residues or less, 30 amino acid residues or less, 25 amino acid residues or less, 20 amino acid residue or less, 15 amino acid residues or less, or 12 amino acid residues or less. In one embodiment, the length of the amino acid linker is, but is not limited to, in the range of 1 amino acid residue to 50 amino acid residues, preferably in the range of 5 amino acid residues to 25 amino acid residues. The linker is preferably a linker that does not adversely affect characteristics of a protein encoded by the nucleic acid of 1) or 2), and a protein encoded by the desired nucleic acid. For example, the linker is preferably a neutral amino acid, such as glycine, serine, and alanine.

In a chimeric protein, the protein encoded by the nucleic acid of 1) or 2) may be bonded to the N terminus side or C terminus side of the protein encoded by the desired nucleic acid. Preferably, the protein encoded by the nucleic acid of 1) or 2) is placed at the N terminus side of the protein encoded by the nucleic acid of the interest. In Example 4, the higher callus formation rate was exhibited when the protein encoded by the nucleic acid of 1) or 2) was present in the N terminus side of the chimeric protein.

The nucleic acid construct (Aspect B) of the present invention can be used for a method of producing a transformed cell or plant body of maize of the present invention and/or the method of introducing a nucleic acid into a cell or plant body of maize of the present invention. The present invention encompasses use of the nucleic acid construct in the method of producing a transformed cell or plant body of maize and/or the method of introducing a nucleic acid in a cell or plant body of maize. The present invention encompasses the nucleic acid construct used for the method of producing a transformed cell or plant body of maize and/or the method of introducing a nucleic acid into a cell or plant body of maize.

### 5. Method of introducing nucleic acid into cell or plant body of maize (Aspect B)

In one embodiment, the present invention relates to a method of introducing a nucleic acid into a cell or plant body of maize (Aspect B).

The method of introducing a nucleic acid into a cell or plant body of maize (Aspect B) of the present invention includes introducing a nucleic acid construct (Aspect B) into maize.

The nucleic acid construct (Aspect B) is as described in "4. Nucleic acid construct (Aspect B)." The method of introducing the nucleic acid construct (Aspect B) into a plant is not particularly limited, and is as described in "3. Method of introducing nucleic acid into cell or plant body of maize." According to the plant transformation method (Aspect B), a fusion protein composed of a protein encoded by the nucleic acid of 1) or 2) and a protein encoded by the desired nucleic acid, i.e., a chimeric protein, is expressed in the plant.

The nucleic acid construct and/or desired nucleic acid may undergo transient expression or stable expression. In one embodiment, without limiting, the nucleic acid construct (Aspect B) undergoes transient expression.

### [Examples]

The present invention will be concretely described through Examples hereinafter, but Examples shall not be construed as limiting the technical scope of the present invention. A person skilled in the art can easily modify or alter the present invention based on the description of the present specification, and such modification and/or alteration is also included in the technical scope of the present invention.

### Example 1 Construction of ZmWOX5bX1 gene transformation vector

A vector for transforming immature embryos of maize with ZmWOX5b was constructed using the Gateway destination vector pDEST1-P35S-Bar (Toda et al., 2019, Supplementary Methods) having Pubi-Iubi-Cm ccdB-Tnos::P35S-bar-T35S on the T-DNA of the binary vector pLC41 (Genbank accession No. LC215698). The Pubi-Iubi-Cm ccdB-Tnos::P35S-bar-T35S is a DNA fragment with an expression unit into which any gene can be inserted by the GATEWAY LR reaction at downstream of the promoter (Pubi) and the first intron (Iubi) of maize ubiquitin 1 gene and a bar gene driven by a 35S promoter (P35S) of Cauliflower mosaic virus as a selectable marker.

A splice variant of ZmWOX5b cDNA of the maize variety B73 (ZmWOX5bX1, Genbank accession No. XM_020550030, SEQ ID NO: 1) was synthesized using the artificial gene synthesis service available from GenScript Japan K.K., and the synthesized splice variant of ZmWOX5b cDNA was inserted between attL1 and attL2 of the Gateway entry vector. The LR reaction was carried out with the obtained entry vector and pDEST1-P35S-Bar to obtain a binary vector pJTM4596 having Pubi-Iubi-ZmWOX5bX1-Tnos::P35S-bar-T35S on the T-DNA.

The binary vector was introduced into Agrobacterium strain LBA4404 together with a booster vector pVGW9 (WO 2014/157541 A1) by electroporation. The resulting LBA4404 was provided for a maize transformation test.

### Example 2 Transformation of immature embryos of maize with ZmWOX5bX1 gene according to known method

Examples 6 and 7 of the patent literature US 8,383,891 B2 discloses that a plasmid DNA having a nucleic acid encoding a WUSCHEL polypeptide is introduced into immature embryos of maize by the particle gun method, callus induction and redifferentiation culture are carried out on a N6 medium for 8 weeks to 10 weeks to obtain transformants. In the above-mentioned patent literature, however, Examples 6 and 7 are described in the present tense, thus it is not certain whether the transformants were actually obtained.

The WUSCHEL polypeptide has an amino acid sequence having 94.8% sequence identity with the sequence of the ZmWOX5b protein (SEQ ID NO: 15) of the present specification. In Example 2, immature embryos of maize variety B73, in which the ZmWOX5bX1 gene was introduced, were cultured according to the method described in the above-mentioned latent literature, and whether transformants could be obtained or not was determined.

For transfer of the ZmWOX5bX1 gene in immature embryos of the maize species B73, the agrobacterium method disclosed in Ishida et al., NATURE PROTOCOLS, 2007, Vol. 2, No. 7, p. 1614-1621 was used. Specifically, immature embryos of the maize variety B73 each having a major axis of from 1.2 mm to 2.0 mm were aseptically taken from a donor plant grown in a greenhouse, and were collected in a 2 mL-volume microcentrifuge tube, in which 2 mL of a liquid medium LS-inf (Ishida et al., 2007) was contained. The centrifuge tube containing the immature embryos was placed in a water bath, and a heat treatment was performed for 4.5 minutes at 46°C. After cooling the centrifuge tube on ice cubes, the immature embryos were washed with the same liquid medium once. Next, centrifuge of 20,000 xg was performed for 10 minutes at 4°C. The Agrobacterium strain prepared in Example 1 was suspended in a LS-inf medium including 100 µM acetosyringone so that absorbance of the resulting suspension was 1.5 at 600 nm. The immature embryos subjected to the heat treatment and the centrifuge treatment were immersed in the Agrobacterium suspension, and were lightly mixed using vortex, followed by standing for 5 minutes. Next, the immature embryos were introduced into an empty 6 cm sterilized Petri dish together with the Agrobacterium suspension, the immature embryos to which the Agrobacterium suspension was attached were placed on a co-culture medium LS-As (Ishida et al., 2007) with the scutellum-side of each immature embryo facing upwards. After culturing for 7 days at 25°C in the dark, the co-cultured immature embryos were placed on a medium (Fromm-1990-N6 P5). The medium was obtained by removing casein from N6 medium, which was the callus-induction medium used in Example 6 of the patent literature US 8,383,891 B2 (Fromm et al. (1990), adding 5 mg/L of phosphinothricin (PPT) (N6 basal inorganic salts (Phytotechnology Laboratories C416) 3.98 g/L, 20 g/L of sucrose, 100 mg/L of myo-inositol, 1.3 mg/L of nicotinic acid , 0.25 mg/L of thiamin-HCl, 0.25 mg/L of pyridoxine-HCl, 0.25 mg/L of calcium pantothenate, 25 mM of proline, 1.5 mg/L of 2,4-D, 5 mg/L of PPT , 8 g/L of agarose, pH 5.8), and adding 250 mg/L of carbenicillin and 100 mg/L of cefotaxime to suppress the growth of Agrobacterium. These immature embryos were cultured for 8 weeks at 25°C in the dark. During the culture, the calluses were introduced onto a fresh Fromm-1990-N6 P5 medium every 2 weeks. Proliferated compact calluses were placed on a medium (Fromm-1990-N6- P5-0.2D) prepared by reducing the 2,4-D concentration of the Fromm-1990-N6 P5 medium to 0.2 mg/L to carry out pre-redifferentiation culture at 25°C. Fourteen days later, the cultured calluses were placed on a medium (Fromm-1990-Reg) prepared by changing the sucrose concentration of the Fromm-1990-N6 P5 medium to 6% and removing 2,4-D to carry out redifferentiation culture at 25°C in light (Control group).

As well as the method disclosed in Example 6 of the patent literature US 8,383,891 B2, the calluses were obtained from any of the following groups through a culture on the Fromm-1990-N6 P5 medium for 8 weeks, then a redifferentiation culture of the obtained calluses was performed. The groups included: a group where a pre-redifferentiation culture was not performed and a redifferentiation culture was performed on the Fromm-1990-Reg (Test group 1); a group where a pre-redifferentiation culture was not performed, and a redifferentiation culture was performed on the Fromm-1990-Reg medium to which 5 mg/M of Zeatin was added as a plant body hormone (Test group 2); and a group where a pre-redifferentiation culture was not performed, and a redifferentiation culture was performed on a LSZ medium (Ishida et al., 2007) composed of MS basal inorganic salts including 5 mg/L of Zeatin (Test group 3). The root cultures of the re-differentiated shoots from Control group and Test group 1 were each performed on a medium (Fromm-1990-Root), which was prepared by substituting the N6 basal inorganic salts (Phytotechnology Laboratories C416, 3.98 g/L) of the Fromm-1990-N6 P5 medium with MS basal inorganic salts (Phytotechnology Laboratories M524, 4.33 g/L) and removing 2,4-D, for 4 weeks at 25°C in light; the root culture of the re-differentiated shoots from Test group 2 was performed on the Fromm-1990-Root medium, to which 0.2 mg/L of IBA was added, for 4 weeks at 25°C in light; the root culture of the re-differentiated shoots from Test group 3 was performed on the LSF medium (Ishida et al., 2007), which was composed of MS basal inorganic salts including 0.2 mg/L of IBA, for 4 weeks at 25°C in light.

Among the immature embryo samples, the count results of the number of immature embryos with which compact calluses were formed on scutellum, the number of immature embryos with which calluses were formed and on which pre-redifferentiation culture or redifferentiation culture was performed, the number of immature embryos with which PPT resistant shoots were re-differentiated, and the number of the immature embryos with which PPT resistant rooting plants were produced are presented in Table 2.

### Table 2 Transformation results of immature embryos of maize with ZmWOX5bX1 gene according to known method

**[Table 2]**

| | Number of immature embryo samples (a) | Callus forming immature embryos | | Number of callus forming immature embryos subjected to pre-redifferentiation or redifferentiation culture (c) | Immature embryos with re-differentiated PPT resistant shoots | | Immature embryos with PPT resistant rooting plants | |
|---|---|---|---|---|---|---|---|---|
| | | Number (a) | Rate (b/a) | | Number (d) | Rate (d/c) | Number (e) | Rate (e/c) |
| Control group | 120 | 28 | 23.3% | 7 | 2 | 28.6% | 0 | 0.0% |
| Test group 1 | | | | 7 | 1 | 14.3% | 1 | 14.3% |
| Test group 2 | | | | 8 | 6 | 75.5% | 1 | 12.5% |
| Test group 3 | | | | 6 | 5 | 83.3% | 1 | 16.7% |

Callus induction was performed on the N6 basal medium for 8 weeks with reference to the known method of transforming immature embryos of maize with the ZmWOX5bXl gene. As a result, among 120 immature embryo samples provided, the number of the immature embryos with which compact calluses were formed was 28 that was equivalent to 23.3%. These immature embryos were subjected to the redifferentiation culture and root culture according to the known method and the methods in which various modifications were added to the known method, but PPT resistant rooting plants were not obtained from the samples according to the known method alone (Control group). Among the methods in which the redifferentiation medium and the rooting medium were modified, moreover, the methods where redifferentiation culture was performed using the medium to which the plant hormone was added (Test groups 2 and 3) exhibited improvements of about 3 times to the control group, namely, the PPT resistant shoot redifferentiation rates were 75.5% and 83.3%, respectively. However, the number of the embryos with which rooting of the shoots was observed was only 1 in each test group.

From the results presented above, it was demonstrated that, when a callus induction culture, redifferentiation culture, and root culture are performed according to the known method (US 8,383,891 B2, control group) in the process of transformation of immature embryos of maize with the ZmWOX5bXl gene, the callus formation rate was low, and a transformed plant body could not be produced. In Example 2, the methods in which the redifferentiation medium and the root medium were modified were tested, but no significant effect was observed in the production efficiency rate of the transformed plant bodies according to any of the methods.

### Example 3 Transformation of maize using ZmWOX5 gene expression vector

Since a transformed plant body was not obtained in the known method in the related art where the immature embryos of the maize were transformed with the ZmWOX5bX1 gene in Example 2, a callus induction period and inorganic salts of media desired for maize transformation using a ZmWOX5bX1 gene were investigated.

For the process of inoculation, co-culture, callus induction culture, and redifferentiation culture, the methods disclosed in Ishida et al. (2007) were referred to. The inoculation and co-culture were performed in the same manner as in Example 2. The immature embryos after the co-culture were placed on the Fromm-1990-N6 P5 medium of Example 2. At the same time, the co-cultured immature embryos were also placed on the medium Fromm-1990-MS P5, in which the inorganic salts of the medium were changed from the N6 basal inorganic salts to MS basal inorganic salts by substituting the N6 basal inorganic salts (Phytotechnology Laboratories C416, 3.98 g/L) of the Fromm-1990-N6 P5 medium with MS basal inorganic salts (Phytotechnology Laboratories M524, 4.33 g/L). These immature embryos were cultured for 5 weeks or 8 weeks at 25°C in the dark. During the culture, the calluses were introduced to a fresh Fromm-1990-N6 P5 medium or Fromm-1990-MS P5 medium every 2 weeks. The proliferated compact calluses or Type I calluses were placed on an LSZ redifferentiation medium (Ishida et al., 2007) including 5 mg/L of PPT, and a redifferentiation culture was performed for 3 weeks to 4 weeks at 25°C under the continuous lighting of 5,000 lx. The re-differentiated shoots were placed on an LSF medium (Ishida et al., 2007) including 5 mg/L of PPT, and a root culture was performed for 4 weeks at 25°C in light.

Among the immature embryo samples provided, the count results of the number of immature embryos with which compact calluses or Type I calluses were formed on scutellum, and the number of the immature embryos with which the PPT resistant shoots were re-differentiated are presented in Table 3. When the callus induction was performed on the N6 medium for 8 weeks according to the known method in which the immature embryos of maize were transformed with the ZmWOX5bX1 gene (control group), similarly to Example 2, redifferentiation of the PPT resistant shoots of the calluses was not observed.

### Table 3 Transformation of immature embryos of Maize B73 using ZmWOX5 gene expression vector

**[Table 3]**

| Test group | Callus induction medium | Callus induction period | Number of immature embryos | | | | | Callus formation rate: b/a | PPT resistant shoot redifferen -tiation efficiency rate: d/c | Production efficiency of pPPT resistant roots: e/c |
|---|---|---|---|---|---|---|---|---|---|---|
| | | | Sample (a) | Callus formation (b) | Callus samples provided for regeneration culture (c) | PET resistant shoot rediffere ntiation (d) | PPT resistant rooting plant (e) | | | |
| Control group | Fromm(1 990) -N6 P5 | 8 weeks | 60 | 19 | 10 | 0 | 0 | 31.7% | 0.0% | 0% |
| 1 | | 5 weeks | | | 9 | 6 | 2 | | 66.7% | 22.2% |
| 2 | Fromm(1 990)-MS P5 | 8 weeks | 71 | 57 | 28 | 16 | 12 | 80.3% | 57.1% | 44.4% |
| 3 | | 5 weeks | | | 29 | 23 | 16 | | 79.3% | 55.2% |

The ratio of the formed calluses relative to the provided immature embryo samples was 31.7% when the callus induction culture was performed on the Fromm-1990-N6 P5 medium, but the rate was significantly increased to 80.3% when the callus induction culture was performed on the Fromm-1990-MS P5 medium. Even with the calluses formed on Fromm-1990-N6 P5 medium, however, redifferentiation of the PPT resistant shoots and rooting were obtained with the obtained calluses, when the callus induction period was 5 weeks (Test group 1). When the callus induction culture was performed on the Fromm-1990-MS P5 medium, even with the callus induction period of 8 weeks, the PPT resistant shoots were re-differentiated from the obtained calluses at the efficiency rate of 57.1%, and the PPT resistant rooting plants were obtained at the efficiency rate of 44.4%. When the callus induction period was shortened to 5 weeks, moreover, the PPT resistant shoot redifferentiation rate and the production rate of the rooting plants were improved, and were 79.3% and 55.2%, respectively.

It became clear from the results above that, when the callus induction culture was performed according to the method known in the related art (US 8,383,891 B2, Control group) to transform the immature embryos of maize with the ZmWOX5bX1 gene, a transformed plant body could not be obtained, but the callus formation rate and the production efficiency rate of the transformed plant bodies significantly improved when the callus induction period was shortened to the period shorter than 8 weeks and/or MS basal inorganic salts were used as inorganic salts of the callus induction medium.

### Example 4 Production of maize variety B73 immature embryo-derived ZmWOX5 transformation callus on MS medium and production_ of suspension-cultured cells

In Example 3, it was demonstrated that calluses were actively formed from the transformed immature embryos of maize with the ZmWOX5bX1 gene using the MS medium. In Example 4, a callus induction culture of the transformed immature embryos of maize B73 with a ZmWOX5bX1 gene was performed using a medium having a composition similar to the Fromm-1990-MS P5 medium used in Example 2, which was formed of the MS medium, and a callus formation effect was studied.

A binary vector for expressing a ZmWOX5bX1-GFP linked gene, in which ZmWOX5bX1 and GFP were linked with 2A peptide, with a promoter of a ubiquitin 1 gene of maize (Pubi) and a first intron (Iubi) was prepared.

ZmWOXSbX1-GSG2A-sGFPS6ST (SEQ ID NO: 3) was prepared by linking a ZmWOX5bX1 gene (SEQ ID NO: 1), a sequence (ggaagcgga) for encoding a GSG spacer, a sequence (5'-CAACTTCTCAACTTTGACTTGCTAAAGTTAGCTGGTGATGTTGAATCTAATCCTGGACCA-3'(SEQ ID NO: 18)) for encoding a foot-and-mouth disease virus (FMDV)-derived 2A peptide (QLLNFDLLKLAGDVESNPGP (SEQ ID NO: 19), Shimatani et al.,(2017)), and sGFPS65T (Chiu et al.,(1996)) by PCR, and was inserted between attL1 and attL2 of the Gateway entry vector. The LR reaction was performed with the obtained entry vector and pDEST1-P35S-Bar to obtain a binary vector pJTM4301 having Pubi-Iubi-ZmWOXSbX1-GSG2A-sGFPS6ST-Tnos::P35S-bar-T35S on T-DNA. The plant cells transformed with pJTM4301 can express a protein in which a peptide other than P of 2A peptide C-end is added to ZmWOZ5bX1 protein, and a protein in which P of 2A peptide C-end and sGFPS65T protein are fused.

The binary vector was introduced into Agrobacterium strain LBA4404 together with a booster vector pVGW9 by electroporation, the resulting LBA4404 was inoculated into immature embryos of maize variety B73 in the same manner as in Example 2 to perform a co-culture. The size of each immature embryo was from 1.0 mm to 1.5 mm, and the heat treatment before inoculation was performed for 3 minutes at 46°C. After the co-culture, the resulting immature embryos were placed on a modified LSD 1.5 medium free from AgNO₃ and PPT (Ishida et al.,(2007), MS base inorganic salts, 100 mg/L of myo-inositol, 0.5 mg/L of nicotinic acid, 1.0 mg/L of thiamin-HCl, 0.5 mg/L of pyridoxine-HCl, 1.5 mg/L of 2,4-D, 20 g/L of sucrose, 0.5 g/L of MES, 0.7 g/L of proline, pH 5.8, 8 g/L of agar, 250 mg/L of carbenicillin, 100 mg/L of cefotaxime), and a callus induction culture was performed for 4 hours at 25°C in the dark. During the culture, the immature embryos were introduced to a fresh modified LSD 1.5 medium, which was identical to the above, every 2 weeks. Among 76 immature embryos to which the callus induction culture was performed on the medium, the number of the immature embryos with which calluses were formed was 62 (81.6%), which was equivalent to the callus formation rate of 80.3% obtained when the callus induction culture of the transformed maize immature embryos with the ZmWox5b was formed on the Fromm-1990-MS P5 medium in Example 3.

The Fromm-1990-MS P5 medium and the modified LSD 1.5 medium were identical in the MS base inorganic salts, 2,4-D concentration, and pH, but were different in the vitamins, proline concentration, and presence of PPT. Among the differences, proline had the largest difference in the amount, and the amounts of proline were 25 mM (2.88 g/L) and 6.1 mM (0.7 g/L), respectively.

The obtained compact calluses were observed under a fluorescence stereo microscope (stereo microscope SZX7 manufactured by Olympus Corporation, lighting for fluorescence observation U-HGLGPS, GFP mirror unit SZX-MGFPA, CCD camera DP73), and the calluses emitting strong fluorescence of GFP were selected and subjected to liquid culture. The calluses (80 mg) were placed in a 200 mL-volume Erlenmeyer flask charged with 40 mL of an LSD 1.5 liquid medium prepared as a liquid medium by removing agar, and were subjected to a shake culture at the speed of 120 rpm at 25°C in the dark. The liquid medium was replaced with a fresh LSD 1.5 liquid medium identical to the above every week, and the liquid culture was continued for 20 weeks. In this manner, suspension-cultured cells composed of compact calluses that did not secrete mucilage whose cell volume would increase approximately twice or three times in 1 week could be produced. The GFP fluorescence of the suspension-cultured cells was observed by the above-described method, and it was confirmed that all the cells emitted fluorescence of GFP. Therefore, it was determined that the suspension-cultured cells were composed of B73 cells transformed with the binary vector used in Example 4.

It was demonstrated from the above-described results that the sufficient callus formation rate was obtained by using the medium formed of the MS basal inorganic salts, when the calluses were obtained from the transformed immature embryos of maize with the ZmWOX5bX1 gene, and no noticeable change was observed in the callus formation rates between the media that differed in medium components, except the MS basal inorganic salts. Moreover, it was demonstrated that the arduously culturable maize variety-derived calluses obtained by overexpressing the ZmWOX5bX1 gene were effective for production of suspension-cultured cells.

### Example 5 Production of suspension-cultured cells from maize variety A188 immature embryo-derived ZmWOX5bX1 transformation calluses

The maize species A188 is a variety that is difficult to form and maintain Type II calluses on a MS medium. To obtain Type II calluses, a method of performing a callus induction culture on a N6 medium to which 6 mM of proline is added, or a method of obtaining calluses from a hybrid variety Hi-II between A188 and B73 have been studied (Todd J. Jones, 2009). In Example 5, a test was performed to form Type II calluses by a callus induction culture of A188 immature embryos, which was transformed with a ZmWOX5bX1 gene, on a MS medium. Moreover, a test was performed to produce suspension-cultured cells using the obtained Type II calluses.

As a control, the LR reaction was performed with a vector and pDEST1-P35S-Bar to prepare a binary vector pJTM3968 having Pubi-Iubi-IGUS-Tnos::P35S-bar-T35S on T-DNA, where the vector was prepared by inserting a GUS gene (IGUS, Ohta et al.,(1990)), in which castor bean catalase introns were placed, between attL1 and attL2 of the Gateway entry vector. The binary vector pJTM3968 having the GUS gene and the binary vector pJTM4596 having the ZmWOX5bX1 gene were each introduced into the Agrobacterium strain LBA4404 together with a booster vector pVGW9, and the resulting vector was used for transformation of A188 immature embryos.

According to the method of Example 2, the obtained Agrobacterium strain was inoculated into A188 immature embryos and a co-culture was performed. Note that, the size of each immature embryo was in the range of 1.5 mm to 2.0 mm, the heat treatment was performed for 4.5 minutes at 46°C, and absorbance of the LS-inf medium, in which Agrobacterium was suspended, at 600 nm was 1.5. After the co-culture, the immature embryos were placed on a modified LSD 1.5-P5 medium (Ishida et al., 2007) which did not include AgNO₃ and to which 5 mg/L of PPT was added, and a callus induction culture was performed at 25°C in the dark. Ten days later, the immature embryos, with which the calluses were enlarged, were placed on a modified LSD 1.5-P10 medium, in which the PPT concentration was increased to 10 mg/L, followed by culturing for 2 weeks at 25°C in the dark. After the co-culture, a callus induction culture was performed for 24 days in total. As a result, Type I calluses were formed on the immature embryos transformed with the GUS gene, but Type II calluses appeared to develop from the entire scutellum of the immature embryos transformed with the ZmWOX5bXl gene. Among them, the Type I calluses obtained by transformation with the GUS gene and the Type calluses obtained by transformation with the ZmWOX5bX1 gene were each selected by 150 mg, and placed in a 200 mL-volume Erlenmeyer flask charged with 40 mL of an LSD 1.5-P5 liquid medium, in which 5 mg/L of PPT was added to the LSD 1.5 liquid medium of Example 4, followed by performing a shake culture at the speed of 120 rpm at 25°C in the dark. The liquid medium was replaced with a fresh medium every week. After performing a shake culture for 2 weeks, cefotaxime in the LSD 1.5-P5 liquid medium was reduced to 50 mg/L and carbenicillin in the LSD 1.5-P5 liquid medium was reduced to 125 mg/L, and a shake culture was further performed for another 2 weeks. Then, a shake culture was further continued in the LSD 1.5-P5 liquid medium in which cefotaxime was reduced to 25 mg/L, and carbenicillin was reduced to 62.5 mg/L. Six weeks later from the initiation of the liquid culture, the Type I callus-derived A188 suspension-cultured cells obtained by transformation with the GUS gene secreted mucilage, thus the culture liquid was clouded. On the other hand, the Type II callus-derived A188 suspension-cultured cells obtained by transformation with the ZmWOX5bX1 gene were composed of the compact calluses that did not secrete mucilage, thus the cell volume was proliferated 2 to 3 times within 1 week.

It became clear from the above-described results that use of the ZmWOX5bX1 gene could yield the Type II calluses from the maize variety that was generally considered as being difficult to obtain Type II calluses on a MS medium. Moreover, it was demonstrated that the obtained Type II calluses were effective for production of suspension-cultured cells.

### Example 6 Nursing effect of ZmWOX5bX1 transformed A188 suspension-cultured cells

A test for determining a nursing effect of the ZmWOX5bX1-transformed A188 suspension-cultured cells produced in Example 5 was carried out.

For processes of separating and culturing fertilized eggs of maize variety B73, the methods disclosed in Koiso et al. (2017) were referred to. Specifically, the silk of the ear of the maize variety B73 grown in greenhouse was artificially pollinated by depositing pollen of the same variety, and the resulting plant body was stored for 12 hours at 25°C, followed by storing for 12 hours at 4°C. Then, nucellus cut pieces each including an embryo sac were excised from ovules of the ear, and were added to 1 mL of a 650 mOsmol/kg mannitol solution. To this, 500 µL of an enzyme mixture solution (a mannitol solution including 1% of cellulase, 0.3% of macerozyme R-10, and 0.05% of pectolyase Y23, pH 7.0), the osmotic pressure of which was adjusted with mannitol, was added. After carrying out an enzymatic reaction for 20 minutes at 32°C, fertilized eggs were separated using a glass rod under an inverted microscope. A 35 mm plastic petri dish, in which only 2 mL of MSO medium was added (control group), and a 35 mm plastic petri dish, in which 2 mL of MSO medium and 180 µL of the liquid medium including ZmWOX5bX1 transformed A188 suspension-cultured cells produced in Example 4 were added (test group), were prepared. Preset VECELL (Vessels PSVC12-10) added with 200 µL of MSO medium was placed in the control group or the test group. The separated fertilized eggs were introduced into the Preset VECELL. A shake culture was performed at the speed of 30 rpm at 26°C in the dark. Cell division was initiated in both the control group and the test group 1 day after the initiation of the shake culture. Ten days later, the fertilized eggs of the control group continued the cell division until growing into a cell mass having a diameter of approximately 0.3 mm, but after that, the growth of the cell was stopped. On the other hand, the fertilized eggs of the test group continued the cell division, and 24 days after the initiation of the culture, a cell mass having a diameter of 1 mm or greater was formed.

As a result, the effect of the ZmWOX5bX1 transformed A188 suspension-cultured cells as nurse cells was confirmed relative to a single cell of maize variety B73 that was difficult to culture.

### Table 4 Nursing effect of ZmWOX5bX1 transformed A188 suspension-cultured cell

**[Table 4]**

| | Nurse culture | Number of fertilized egg samples (a) | Number of cell masses in size of 1 mm or larger (b) | (b/a: %) |
|---|---|---|---|---|
| Control group | None | 5 | 0 | 0% |
| Test group | ZmWOX5b transformed A188 suspension-cultured cells | 7 | 5 | 71.4% |

### Example 7 Construction of vector for transformation of various maize-derived WOX genes

Binary vectors for overexpressing various WOX genes derived from maize, and as a comparative control, a vector with reference to a sequence of a known binary vector PHP35648 (Lowe et al., 2016) using a ZmWUS2 gene and a ZmBBM gene that were known to be successful in transformation of the arduously culturable maize variety were constructed.

Vectors for transforming immature embryos of maize with ZmWUS2, ZmWOX2a, ZmWOX4, ZmWOX11, ZmNS2, and ZmBBM2, respectively, were constructed using the Gateway destination vector pDEST1-P35S-Bar having Pubi-Iubi-Cm ccdB-Tnos::P35S-bar-T35S on T-DNA of the binary vector pLC41 (Toda et al., 2019 Supplementary Methods).

A ZmWOX2a cDNA sequence (SEQ ID NO: 4), ZmWOX4 cDNA sequence (SEQ ID NO: 5), ZmWOX11 cDNA sequence (SEQ ID NO: 6), ZmWUS2 cDNA sequence (Genbank accession No. EA275154, SEQ ID NO: 7), ZmNS2 cDNA sequence (SEQ ID NO: 8), ZmBBM2 (Genbank accession No. XM008676474, SEQ ID NO: 9) were synthesized through the artificial gene synthesis service available from GenScript Japan K.K., and were each inserted between attL1 and attL2 of the Gateway entry vector. The LR reaction was carried out with each of the obtained entry vectors and pDEST1-P35S-Bar to obtain a binary vector pJTM4556 having Pubi-Iubi-ZmWOX2a-Tnos::P35S-bar-T35S on T-DNA, a binary vector pJTM4690 having Pubi-Iubi-ZmWOX4-Tnos::P35S-bar-T35S, a binary vector pJTM4584 having Pubi-Iubi-ZmWOX11-Tnos::P35S-bar-T35S, a binary vector pJTM4548 having Pubi-Iubi-ZmWUS2-Tnos::P35S-bar-T35S, a binary vector pJTM4557 having Pubi-Iubi-ZmNS2-Tnos::P35S-bar-T35S, and a binary vector pJTM4408 having Pubi-Iubi-ZmBBM-Tnos::P35S-bar-T35S. Within the pJTM4408, moreover, Pnos-ZmWUS2-Tpin (SEQ ID NO: 10) synthesized also using the artificial gene synthesis service available from GenScript Japan K.K. was further inserted in the restriction enzyme site AscI to provide a binary vector pJTM4522 having Pubi-Iubi-ZmBBM-Tnos::P35S-bar-T35S::Pnos-ZmWUS2-Tpin on the T-DNA.

The binary vector was introduced into Agrobacterium strain LBA4404 together with a booster vector pVGW9 by electroporation, and the resulting LBA4404 was provided for a maize transformation test.

### Example 8 Transformation of maize immature embryos with various maize-derived WOX genes

In order to compare and study the effects of various WOX genes derived from maize in callus formation and transformation of maize, a test of transforming immature embryos of the arduously culturable maize variety B73 with the WOX gene expression binary vectors produced in Examples 1 and 7 was carried out.

The Agrobacterium strain LBA4404 produced in Examples 1 and 7 was used, and was inoculated into immature embryos of maize variety B73 in the same manner as in Example 2 to perform a co-culture. The size of each immature embryo was from 0.8 to 2.0 mm. If a large number of the immature embryos each having a major axis of 0.8 mm to 1.5 mm was included, a heat treatment was performed for 3 minutes at 46°C before inoculation. If a large number of the immature embryos each having a major axis of 1.5 mm to 2.0 mm was included, a heat treatment was performed for 4.5 minutes at 46°C before inoculation. After the co-culture, the resulting embryos were placed on a modified LSD 1.5 medium (Ishida et al., 2007) that did not include AgNO₃ nor PPT, and a callus induction culture was performed for 5 weeks at 25°C in the dark. During the callus induction culture, the calluses were introduced to a fresh modified LSD 1.5 medium every 2 weeks. The proliferated compact calluses or Type I calluses were placed on an LSZ (Ishida et al., 2007) redifferentiation medium including 5 mg/L of PPT, and a redifferentiation culture was performed for 3 weeks to 4 weeks at 25°C under the continuous lighting of 5,000 lx. The calluses, with which shoots were re-differentiated, were placed on an LSF (Ishida et al., 2007) rooting medium including 5 mg/L PPT, and a root culture was performed for 4 weeks at 25°C under continuous lighting of 5,000 lx.

Among the immature embryo samples provided, the count results of the number of immature embryos with which compact calluses or Type I calluses were formed on scutellum, the number of immature embryos with which PPT resistant shoots were re-differentiated, and the number of immature embryos with which PPT resistant rooting plants were obtained are presented in Table 5.

### Table 5 Results of transformation of immature embryos of maize with various WOX genes derived from maize

**[Table 5]**

| Gene | Immature embryos | | | | Callus formation efficiency rate (b/a) | PET resistant shoot redifferentiation efficiency rate (c/a) | Production efficiency rate of PPT resistant rooting plants (d/a) |
|---|---|---|---|---|---|---|---|
| | Number of samples (a) | Callus formation number (b) | Number of re-differentiated PET resistant shoots (c) | Number of PET resistant rooting plants (d) | | | |
| IGUS | 55 | 0 | 0 | 0 | 0.0% | 0.0% | 0.0% |
| ZmWOX2a | 360 | 41 | 9 | 0 | 11.4% | 2.5% | 0.0% |
| ZmWOX11 | 300 | 3 | 0 | 0 | 1.0% | 0.0% | 0.0% |
| ZmWOX5bX1 | 672 | 467 | 282 | 58 | 69.8% | 40.9% | 8.6% |
| ZmWus2 | 269 | 142 | 48 | 11 | 52.8% | 17.8% | 4.1% |
| ZmNS2 | 260 | 33 | 0 | 0 | 12.7% | 0.0% | 0.0% |
| ZmWus2-ZmBBM2 | 157 | 135 | 52 | 0 | 86.0% | 33.1% | 0.0% |

The calluses were formed from the B73 immature embryos transformed with the maize-derived WOX genes at various efficiency rates, whereas calluses were not obtained from B73 immature embryos in case of transformation with the IGUS gene. The genes achieving the high callus formation efficiency rates and PPT resistant shoot redifferentiation rates were ZmWux2-ZmBBM2, ZmWOX5bX1, and ZmWus2, all of which achieved formation of calluses at the efficiency rate of 50% or greater relative to the immature embryo samples provided, and achieved redifferentiation of the PPT resistant shoots at the efficiency rate of 15% or greater. In the case of the transformation with ZmWux2-ZmBBM2, however, rooting did not occur from the re-differentiated PPT resistant shoots, thus a transformed plant body was not obtained. This matched with the report from Lowe et al. (2016) where, when maize was transformed with ZmWus2-ZmBBM, it was difficult to obtain a transformed plant body from calluses formed of transformed cells with the above-mentioned 2 genes as they were, and it was necessary to remove these 2 genes from the cells during the culture. In the case of the transformation with ZmWOX5bX1 or ZmWus2, on the other hand, rooting occurred from the re-differentiated PPT resistant shoots, and transformed plant bodies could be obtained at the efficiency rates 8.6%, and 4.1%, respectively, from the immature embryo samples provided. On the other hand, other maize-derived WOX genes achieved the low callus formation efficiency rates, and only formed calluses at the efficiency rate of lower than 20% relative to the immature embryo samples.

It became clear from the results above that, among the maize-derived WOX genes provided in Example 8, the ZmWOX5 gene achieved the highest callus formation efficiency rate from the maize and the highest production efficiency rates for PPT resistant shoots and rooting plants. The results indicate that use of the ZmWOX5 gene in the transformation of maize is extremely effective for production of a transformed plant body from calluses formed of transformed cells of an arduously culturable maize variety, which has been impossible by the known method using ZmWus2-ZmBBM. As the ZmWOX5 gene was used, moreover, the length of T-DNA on the binary vector could be shortened by approximately half (T-DNA length: 4,577 bp) compared to the known method using ZmWus2-ZmBBM (T-DNA length: 9,114 bp). This suggests that use of the ZmWOX5 gene can contribute to lower the difficulty of construction of a vector or transformation.

Meanwhile, according to the results of Example 8, the number of the obtained PPT resistant rooting plants was 58 among 467 that was the number of the B73 calluses obtained by transformation with ZmWOX5bX1, and the efficiency rate remained 12.4%. This result was largely lower than 55.2%, which was the result of Test group 3 in Example 3. The difference between the B73 transformation method of Example 3 and that of Example 8 was whether the Fromm-1990-MS P5 medium was used, or the modified LSD 1.5 medium was used as the callus induction medium. In Example 4, the difference between the composition of the Fromm-1990-MS P5 medium and the composition of the modified LSD 1.5 medium did not demonstrate a large difference in the callus formation effect. According to the results of Example 8, however, it was demonstrated that the Fromm-1990-MS P5 medium functioned more effectively in the rooting effect than the modified LSD 1.5 medium. The Fromm-1990-MS P5 medium and the modified LSD 1.5 medium had the same MS basal inorganic salts, 2,4-D concentration, and pH, but were different in the vitamins, proline concentration, and presence of PPT. Among the differences, the largest difference in the amount was proline, and the Fromm-1990-MS P5 medium and the modified LSD 1.5 medium included the proline at the concentrations of 25 mM (2.88 g/L) and 6.1 mM (0.7 g/L), respectively. This indicates that it is effective to set the proline content of the callus induction medium higher than 6.1 mM to efficiently obtain transformed shoots, and transformed plant bodies having re-differentiated roots from the transformed immature embryos of maize with the ZmWOX5bX1 gene.

### Example 9 Construction of transformation vectors of WOX5 genes of various plant varieties

Binary vectors for overexpressing WOX5 gene homologue derived from various plant varieties were constructed.

Using the Gateway destination vector pDEST1-P3SS-Bar (Toda et al., 2019 Supplementary Methods), which had Pubi-Iubi-Cm ccdB-Tnos::P35S-bar-T35S on T-DNA of the binary vector pLC41, vectors for transforming immature embryos of maize with wheat WOX5 (TaWOX5), rice WOX5 (OsWOX5), paralogue of maize WOX5b (ZmWOX5a), and ZmWOX5b obtained from regular gene splicing of maize WOXSbX1, respectively, were constructed.

The cDNA (SEQ ID NO: 11) of wheat TaWOX5 disclosed in WO2018/224001 A1, cDNA (synonym: OsWOX9, LOC_Os01g63510.1, SEQ ID NO: 12) of the rice variety, *Oryza sative L.,* OsWOX5 disclosed in WO2018/224001 A1, cDNA (Transcript ID: AFW84527, Gramene ID: GRMZM2G478396_T01, SEQ ID NO: 13) of maize variety B73 ZmWOX5a, and cDNA (Transcript ID: DAA56787, Gramene ID: GRMZM2G116063_T01, SEQ ID NO: 14) of maize variety B73 ZmWOX5b were synthesized using the artificial gene synthesis service available from GenScript Japan K.K., and each of the synthesized genes was inserted between attL1 and attL2 of the Gateway entry vector. The LR reaction was carried out with each of the obtained entry vectors and pDEST1-P35S-Bar to obtain a binary vector pJTM4549 having Pubi-Iubi-TaWOX5-Tnos::P35S-bar-T35S on T-DNA, a binary vector pJTM4822 having Pubi-Iubi-OsWOX5-Tnos::P35S-bar-T35S on T-DNA, a binary vector pJTM4593 having Pubi-Iubi-ZmWOX5a-Tnos::P35S-bar-T35S on T-DNA, and a binary vector pJTM4726 having Pubi-Iubi-ZmWOX5b-Tnos::P35S-bar-T35S on T-DNA.

The binary vector was introduced into Agrobacterium strain LBA4404 together with a booster vector pVGW9 by electroporation, and the resulting LBA4404 was provided for a maize transformation test.

### Example 10 Transformation of immature embryos of maize with WOX5-related gene derived from various plant varieties

In order to compare and study the effects of WOX5 gene homologue derived from various plant varieties in callus formation and transformation of maize, a test was carried out to transform immature embryos of the arduously culturable maize variety B73 using the binary vector pJTM4596 produced in Example 1 or the binary vectors for expressing 4 types of WOX5 genes produced in Example 9.

The inoculation, co-culture, callus induction culture, and redifferentiation culture of immature embryos of maize variety B73 were performed in the same manner as in Example 8.

Among the immature embryo samples provided, the count results of the number of immature embryos with which compact calluses or Type I calluses were formed on scutellum, the number of immature embryos with which PPT resistant shoots were re-differentiated, and the number of immature embryos with which PPT resistant rooting plants were obtained are presented in Table 6 below.

### Table 6 Results of transformation of immature embryos of maize with WOX5 genes of various plant varieties

**[Table 6]**

| Transcription factor used for overexpression | Immature embryo | | | Callus formation efficiency rate (b/a) | PPT resistant shoot redifferentiation efficiency rate (c/a) |
|---|---|---|---|---|---|
| | Number of samples (a) | Callus formation number (b) | Number of re-differentiated PPT resistant shoots (c) | | |
| TaWOX5 | 578 | 231 | 136 | 40.0% | 23.5% |
| OsWOX5 | 154 | 81 | 42 | 52.6% | 27.3% |
| ZmWOX5a | 358 | 72 | 37 | 20.1% | 10.3% |
| ZmWOX5b | 49 | 31 | 21 | 63.3% | 42.9% |
| ZmWOX5bX1 | 230 | 163 | 87 | 70.9% | 37.8% |

In the tests where ZmWOX5b and ZmWOX5bX1 were introduced, the callus formation efficiency rates and the redifferentiation efficiency rates of PPT resistant shoots relative to the B73 immature embryos were significantly high, and were 60% or greater, and 35% or greater, respectively. It became clear from the results as described above that the ZmWOX5b gene induced formation of calluses from explants of the maize varieties, which did not generally form calluses, regardless of embodiments of splicing, and had an effect of regenerating shoots. Moreover, ZmWOX5a, which was a paralogue of the maize WOX5b gene, did not exhibit the same effects on both the callus formation efficiency rate and the redifferentiation efficiency rate of PPT resistant shoots, as with the effects of ZmWOX5b and ZmWOX5bX1, and the callus formation efficiency rate and the redifferentiation efficiency rate of PPT resistant shoots were 20.1% and 10.3%, respectively. The TaWOX5 and OsWOX5, which were WOX5 genes of plant varieties other than maize, also did not exhibit the same effects on both the callus formation rate and the redifferentiation efficiency rate of PPT resistant shoots as with the effects of the ZmWOX5b and ZmWOX5bX1.

The homology between the amino acid sequences of the WOX5 genes is presented in Table 7 below.

### Table 7 Homology of various transcription factors at amino acid level

**[Table 7]**

| Transcription factor | Homology of proteins (%) | | | | |
|---|---|---|---|---|---|
| | TaWOX5 | OsWOX5 | ZmWOX5a | ZmWOX5b | ZmWOX5bX1 |
| TaWOX5 | | 79 | 66 | 74 | 71 |
| OsWOX5 | 79 | | 71 | 82 | 79 |
| ZmWOX5a | 66 | 71 | | 74 | 77 |
| ZmWOX5b | 74 | 82 | 74 | | 94 |
| ZmWOX5bX1 | 71 | 79 | 77 | 94 | |

It was understood from the results of Tables 6 and 7 that there was no correlation between the callus formation efficiency rate and redifferentiation efficiency rate of PPT resistant shoots with homologue of the WOX5 amino acid sequences. From the amino acid sequences of eWOX5 genes of various plant varieties, the results that ZmWOX5b and ZmWOX5bX1 had the highest callus formation efficiency rate and the highest redifferentiation efficiency rate of PPT resistant shoots could not be predicted.

### Example 10 Construction of ZmWOX5 gene expression vectors with various promoters

Binary vectors for expressing a ZmWOX5b gene with various promoters were constructed.

Expression of ZmWOX5bX1 was attempted using, as a constitutive expression promoter, PubiIubi composed of a promoter of a ubiquitin 1 gene of maize and a sequence of the first intron, and P35SIcat (SEQ ID NO: 16) having a sequence including a 35S promoter of Cauliflower mosaic virus and a castor bean catalase intron downstream of the 35S promoter; and as a tissue-specific promoter, a phospholipid transferase promoter (Lowe et al., (2018), PPLTP, SEQ ID NO: 17) that specifically functioned in leaves, embryos, and calluses of maize.

Removal and ring-opening of the Pubi-Iubi sequence were carried out by digesting the binary vector pJTM4596 of Example 1 with a restriction enzyme XbaI, and P35SIcat and PPLTP were each inserted into the digested site. In this manner, a binary vector pJTM4806 having P35SIcat-ZmWOX5bX1-Tnos::P35S-bar-T35S on T-DNA and a binary vector pJTM4850 having PPLTP-ZmWOX5bX1-Tnos::P35S-bar-T35S on T-DNA were obtained.

Each of the binary vectors was introduced into Agrobacterium strain LBA4404 together with a booster vector pVGW9 by electroporation, and the resulting LBA4404 was provided for a maize transformation test.

### Example 11 Transformation of immature embryos of maize using gene ZmWOX5 expression vector with various promoters

In order to observe effects of ZmWOX5 gene expression with various promoters in callus formation and transformation of maize, a test was carried out to transform immature embryos of the arduously culturable maize variety B73 using the binary vector pJTM4596 produced in Example 1 and two types of the ZmWOX5b gene expression binary vectors produced in Example 10.

The inoculation, co-culture, callus induction culture, and redifferentiation culture of immature embryos of maize variety B73 were performed in the same manner as in Example 8.

Among the immature embryo samples provided, the count results of the number of immature embryos with which compact calluses or Type I calluses were formed on scutellum, the number of immature embryos with which PPT shoots were re-differentiated, and the number of immature embryos with which PPT resistant rooting plants were obtained are presented in Table 8 below.

### Table 8 Results of transformation of immature embryos of maize using ZmWOX5 gene expression vector with various promoters

**[Table 8]**

| Promoter | Immature embryo | | | Callus formation efficiency rate (b/a) | Redifferentiation efficiency rate of PPT resistant shoots (c/a) |
|---|---|---|---|---|---|
| | Number of samples (a) | Callus formation number (b) | Number of re-differentiated PPT resistant shoots (c) | | |
| Pubilubi | 142 | 114 | 53 | 80.3% | 37.3% |
| P35SIcat | 146 | 117 | 44 | 80.1% | 30.1% |
| PPLTP | 136 | 116 | 93 | 85.3% | 68.4% |

As a result, very high values were also achieved in the case where ZmWOX5bX1 was expressed under the control of P35SIcat or PPLTP. Specifically, the calluses formation efficiency rates were 80% or greater, and the redifferentiation efficiency rates of PPT resistant shoots were 30% or greater. It became clear from the results above that the ZmWOX5b gene contributed to formation of calluses or production of transformants in transformation of maize not only in high constitutive expression with PubiIubi, but the ZmWOX5b gene also exhibited the same effects when the expression was regulated by other constitutive promoters or tissue-specific promoters.

### Example 12 Morphological comparison between maize transformants produced using ZmWOX5b gene expression vector with various promoters

The morphologies of the maize transformants produced in Example 11 were observed to determine whether there was a significant abnormality (abnormality of the phenotype) in the morphology of the transformant depending on the promoter used with the ZmWOX5bXI gene.

A root culture of the calluses obtained in Example 11, with which PPT resistant shoots were re-differentiated, was performed in the same manner as in Example 8 to produce rooting plants having PPT resistant roots. The PPT resistant rooting plants were determined as transformants. The transformants were planted in soil, and were cultivated for 2 months in greenhouse, followed by carrying out morphology observation. A plant that demonstrated either or both a case where a stock did not grow straight and a case where shrinkage of leaves was observed was determined as a plant having an abnormal phenotype; a plant body that demonstrated none of the above-described cases was determined as a morphologically regular plant. Among the number of plants observed, the count results of the regular plants and the abnormal plants are presented in Table 9 below.

In the present example, moreover, a fertility rate was also determined. The observed plants included: plants where stocks did not grow straight (and/or shrinkage of leaves was observed) and reproductive tissues were not differentiated (resulting in no fertility); plants where stocks did not grow straight (and/or shrinkage of leaves was observed) but reproductive tissues were differentiated and fertilized; plants where stocks grew straight and shrinkage of leaves did not observed, but reproductive tissues were not differentiated (resulting in no fertility); plants where cross-fertilization was carried out but resulting in no fertility. The number of plants that ultimately seeded (were fertilized) was counted as the fertilized plants.

### Table 9 Morphology observation results of maize transformants produced using ZmWOX5b gene expression vector with various promoters

**[Table 9]**

| Promoter | Number of observed plants (a) | Regular morphology | | Abnormal morphology | | Fertility | |
|---|---|---|---|---|---|---|---|
| | | Number of plants (b) | Rate: b/a | Number of plants (c) | Rate: c/a | Number of plants (d) | Rate: d/a |
| Pubilubi | 12 | 4 | 33.3% | 8 | 66.7% | 1 | 8.3% |
| P35SIcat | 13 | 13 | 100% | 0 | 0.0% | 8 | 61.5% |
| PPLTP | 11 | 2 | 18.2% | 9 | 81.8% | 5 | 45.5% |

As presented in Table 9, the maize transformants produced using the vector that expressed ZmWox5bXI under the control of PubiIubi or the vector that expressed ZmWox5bXI under the control of PPLTP included plants having the abnormal phenotype at the rates of 66.7%, and 81.8%, respectively. When the vector that expressed ZmWox5bXI under the control of P35SIcat was used, on the other hand, plants having the abnormal phenotype were not produced. Moreover, it was confirmed that the plants judged as plants of regular morphology had fertility.

The above-described results indicated that, in transformation of maize using the ZmWox5b gene, the abnormality of the phenotype of plants was caused under the control of the known constitutive promoter PubiIubi, or in transformation of maize using other morphogenetic genes under the known tissue-specific promoter PPLTP, but the abnormality of the phenotype could be inhibited when P35SIcat was used. Moreover, the results proved that the conventional insight where it was difficult to obtain a transformed plant body of regular morphology from constitutively overexpressed cells with a morphogenetic gene, could be noticeably overturned by using an appropriate promoter.

### Example 13 Transformation of maize using ZmWOX5b gene expression vector with 35S promoter, and morphology observation of transformants

A test was conducted to determine whether an abnormal phenotype inhibition effect on transformation of maize with ZmWox5bXI exhibited in Example 12 was attributed to the 35S promoter (P35S) of the Cauliflower mosaic virus, and the abnormal phenotype inhibition effect could be exhibited not only with the ZmWox5bXI gene but also with a ZmWox5b gene.

First, a vector for expressing ZmWox5b with P35SIcat, and a vector for expressing ZmWox5b with only P35S without Icat were constructed. The binary vector pJTM4726 of Example 9 was digested with restriction enzyme XbaI to remove and cause ring-opening of the Pubi-Iubi sequence. In the digested site, each of P35SIcat and P35S was inserted. As a result, a binary vector having P35SIcat-ZmWOX5b-Tnos::P35S-bar-T35S on T-DNA, and a binary vector having P35S-ZmWOX5b-Tnos::P35S-bar-T35S on T-DNA were obtained. Each binary vector was introduced into Agrobacterium strain LBA4404 together with a booster vector pVGW9 by electroporation, and the resulting LBA4404 was provided for a transformation test of maize. As a control, the binary vector pJTM4806 having P35SIcat-ZmWOX5bX1-Tnos::P35S-bar-T35S on T-DNA, which was used in Example 11, was also provided for a transformation test.

Using the above-described Agrobacterium strain, inoculation, co-culture, callus induction culture, redifferentiation culture, and root culture of immature embryos of maize variety B73 were performed in the same manner as in Example 8. Note that, a medium having 5 mg/L of PPT was used as the modified LSD 1.5 medium. A plant in which PPT resistant shoots and roots were re-differentiated was determined as a transformant, and a transformation efficiency rate (a ratio of immature embryos that produced transformants relative to the immature embryo samples provided) was calculated. The transformation efficiency rates of P35SIcat-ZmWOX5b, P35S-ZmWOX5b, and P35SIcat-ZmWOX5bXI were 27.6%, 20.7%, and 31.0%, respectively. The obtained transformants were each planted in soil, and were cultivated for 2 months in greenhouse, followed by performing morphology observation. The morphology determination results according to the indexes of Example 12 are presented in Table 10.

### [Table 10]

**Table 10 Morphology observation results of transformed maize plants in which ZmWox5b was expressed under the control of P35SIcat or P35S**

| Promoter | Number of plants observed (a) | Regular morphology | | Abnormal morphology | |
|---|---|---|---|---|---|
| | | Number of plants (b) | Rate: b/a | Number of plants (d) | Rate: c/a |
| P35SIcat-ZmWox5b | 8 | 7 | 87.5% | 1 | 12.5% |
| P35S-ZmWox5b | 5 | 5 | 100% | 0 | 0.0% |

The morphologies of the maize transformants produced by expressing the ZmWox5b gene using the P35SIcat or P35S were regular morphology at the high rate, 85% or higher, relative to the number of observed plants. It was demonstrated from the results above that P35S achieved transformation of the arduously culturable maize variety with the ZmWox5b gene and ZmWox5bXI gene at a high efficiency rate regardless of the presence of an intron having a gene expression promotion effect, such as Icat, and the produced transformants inhibited the abnormality of the phenotype. Moreover, corn silk was differentiated in all of the plants judged as having regular morphology. Furthermore, the fertilized plants were confirmed.

### Example 14 Production of maize transformant having predetermined gene using ZmWox5b gene

A transformant of an arduously culturable maize variety B73 having a predetermined gene different from a ZmWox5b gene was produced using a ZmWox5b. In Example 14, a transformation test was performed using a binary vector, in which a predetermined gene was placed on different T-DNA from the T-DNA where the ZmWox5b was present, as a method capable of separating the ZmWox5b and the predetermined gene in a progeny plant of a transformant.

### (1) Production of binary vector pVS-ZmWox5b

First, RB to LB in pLC41 was amplified using pLC41 as a template by PCR using the following primer pair.

Forward primer to which SpeI site was added (SEQ ID NO: 20) 5'-GACTAGTCGACAAGCAGATCACGCTTTTCGAC-3'
Reverse primer (SEQ ID NO: 21) 5'-GACTAGTCTCCAAGAGACGGTTACACAAACGG-3'

The amplified product was digested with SpeI, and inserted into pGW, which had been ring-opened with XbaI in advance (WO2014/157541 A1) (Patent Literature 4). As a result, pGW-T-DNA where the gentamicin resistance gene was present next to T-DNA, was obtained. Subsequently, a section of the sequence from the gentamicin resistance gene to T-DNA was amplified using the pGW-T-DNA as a template by PCR using the following primer pair.

Forward primer formed of a sequence in which an EcoRV site was added upstream of the gentamicin resistance gene (SEQ ID NO: 22) 5'-GATATCCCGGGTTGACATAAGCCTGTT-3'
Reverse primer formed of a downstream sequence of LB of T-DNA (SEQ ID NO: 23)
   5'-CTCCAAGAGACGGTTACACAAAC-3'

The amplified product was inserted into the ScaI site of pCAMBIA1201. As a result, a gentamicin resistant binary vector pVS-Gm-T-DNA having a pVS-based skeleton was obtained.

The spectinomycin resistance gene was amplified using pSB11 (AB027256) as a template by PCR using the following primer pair.

Forward primer formed of the 5' end side sequence of spectinomycin resistance gene (SEQ ID NO: 24)
   5'-AGCTCTCTAACGCTTGAGTTAAGC-3'
Reverse primer formed of the 3' end side sequence of spectinomycin resistance gene (SEQ ID NO: 25)
   5'-CGTTTCGGTGATGACGGTGAA-3'

The amplified product was digested with EcoRV in advance, and then was inserted into DNA that was obtained by removing the gentamicin resistance gene from the previously produced pVS-Gm-T-DNA to thereby obtain a binary vector pVS-Sp-T-DNA having a pSV-based skeleton. Moreover, P35SIcat-ZmWox5b-Tnos was introduced into the HindIII site of the pVS-Sp-T-DNA, and Pubi-Iubi-DsRed2-Tnos was introduced into the KpnI site of the pVS-Sp-T-DNA to produce pVS-ZmWox5b.

### (2) Production of binary vector pLC41-GFP

To use a sGFPS65T gene as the intended gene, a binary vector for expressing a sGFPS65T gene was produced. The LR reaction was carried out with a vector, in which a sGFPS65T gene was inserted between attL1 and attL2 of the Gateway entry vector, and pDEST1-P35S-Bar to produce a binary vector pLC41-GFP having Pubi-Iubi-sGFPS65T-Tnos::P35S-bar-T35S on T-DNA.

### (3) Transformation

Three binary vectors, the pVS-ZmWox5b produced in (1), the pLC41-GFP produced in (2), and pJTM3968 (Example 5, Pubi-Iubi-IGUS-Tnos::P35S-bar-T35S was placed on T-DNA), were each introduced together with a booster vector pVGW9 (Patent Literature 4) into separate Agrobacterium strain LBA4404, to prepare pVS-ZmWox5b/LBA4404, pLC41-GFP/LBA4404, and pJTM3968/LBA4404, respectively. Moreover, a combination of pVS-ZmWox5b and pLC41-GFP, and a combination of pVS-ZmWox5b and pJTM3968 were each introduced into LBA4404 in the same manner as with pVGW9, to thereby prepare pVS-ZmWox5b+pLC41-GFP/LBA4404, and pVS-ZmWox5b+pJTM3968/LBA4404, respectively.

For the maize transformation test, strains where pVS-ZmWox5b/LBA4404 was combined with pLC41-GFP/LBA4404 (Test 1) or pJTM3968/LBA4404 (Test 2), a single strain of pVS-ZmWox5b+pLC41-GFP/LBA4404 (Test 3), and a single strain of pVS-ZmWox5b+pJTM3968/LBA4404 (Test 4) were used.

Using the above-mentioned Agrobacterium virus, inoculation, co-culture, callus induction culture, redifferentiation culture, and root culture of immature embryos of maize variety B73 were performed in the same manner as in Example 13. Note that, in Test 1 and Test 2, pVS-ZmWox5b/LBA4404 and pLC41-GFP/LBA4404, or pJTM3968/LBA4404 were combined and used as inoculants so that the OD600 ultimately reached 1.25 and 1.75, respectively. Moreover, pJTM3968/LBA4404 was used as an inoculant of a control.

After the root culture, genome DNAs were extracted from leaves of the plants, in which the PPT resistant shoots and roots were re-differentiated, and PCR was carried out to detect the GFP gene and GUS gene. As a result, the plant from which the GFP gene or GUS gene was detected was determined as a transformant. The count results of the number of immature embryos with which the transformants were obtained are presented in Table 11.

### [Table 11]

**Table 11 Results of transformation of maize with predetermined gene using ZmWox5b**

| | Agrobacterium strain | Predetermined gene | Number of immature embryos | | Trans formation efficiency rate (b/a) |
|---|---|---|---|---|---|
| | | | Provided samples (a) | Trans formants (b) | |
| Test 1 | pVS-ZmWox5b/LBA4404 pLC-GFP/LBA4404 | GFP | 30 | 25 | 83.3% |
| Test 2 | pVS-ZmWox5b/LBA4404 pJTM3968/LBA4404 | GUS | 36 | 29 | 80.6% |
| Test 3 | pVS-ZmWox5b+pLC-GFP/LBA4404 | GFP | 37 | 19 | 51.4% |
| Test 4 | pVS-ZrrWox5b+pJTM3968/LBA4404 | GUS | 38 | 22 | 57.9% |
| Control | pJTM3968/LBA4404 | GUS | 47 | 0 | 0.0% |

The transformants could not be obtained in Control group where the ZmWox5b gene was not used. In the test where the ZmWox5b gene was used, on the other hand, the transformants were obtained at the transformation efficiency rate of 80% or greater when the Agrobacterium strain including the ZmWox5b gene and the GFP gene was used (Test 1), or the strain in which the Agrobacterium strain including the GUS gene was mixed was used (Test 2), and the transformation efficiency rate of 50% or greater when the Agrobacterium strain including the ZmWox5b gene and the GFP gene was used (Test 3), or the single Agrobacterium strain including both genes of the GUS gene was used (Test 4). As the transformation efficiency rate of the arduously culturable maize variety B73 known in the related art, Lowe et al. (2018) reported the highest transformation efficiency rate, and the transformation efficiency rates from the 3 repetition tests were 9%, 13%, and 50%, respectively.

In Example 14, in any of the tests using the ZmWox5b gene, the transformation efficiency rates were significantly higher than the transformation efficiency rates known in the related art. Therefore, the transformation with the ZmWox5b gene can be applied for an arduously culturable maize variety, and is extremely effective compared to the transformation technique known in the related art (control).

### Example 15 Transformation of maize using various MS media

Callus induction was carried out using each of media including the MS basal inorganic salts at various concentrations to produce a maize transformant having a ZmWox5b gene.

Media of various compositions used for the callus induction was prepared as described in Table 12. For the N6 basal inorganic salts, which were 10×N6 major and 10×N6 minor, Hiei et al. (2008) (NPL 20,) was referred to.

### [Table 12]

**Table 12 Media including various concentrations of MS basal inorganic salts**

| /L Medium | 1×MS | 0.8×MS | 0.5×MS | 1×MS+0.5×N6 | 0.5×MS+0.5×N6 | 1×6N |
|---|---|---|---|---|---|---|
| 10×LS major salts | 100 mL | 80 mL | 50 mL | 100 mL | 50 mL | |
| 10×N6 major salts | | | | 50 mL | 50 mL | 100 mL |
| 100×LS minor salts | 10 mL | 8 mL | 5 mL | 10 mL | 5 mL | |
| 100×N6 minor salts | | | | 5 mL | 5 mL | 10 mL |
| 100×Fe | 10 mL | 8 mL | 5 mL | 10 mL | 10 mL | 10 mL |
| 100×LS vitamin | 10 mL | | | | | |
| 100 mg/L, 2,4-D | 15 mL | | | | | |
| Proline | 0.7 g | | | | | |
| MES | 0.5 g | | | | | |
| Agar | 8 g | | | | | |
| 20 mg/mL PPT | 0.25 mL | | | | | |
| 0.25 g/mL cefotaxime | 0.4 mL | | | | | |
| 0.25 g/mL carbenicillin | 1.0 mL | | | | | |

| | | | | | | |
|---|---|---|---|---|---|---|
| pH 5.8 | | | | | | |

Inoculation, co-culture, callus induction culture (5 weeks), redifferentiation culture, and root culture of immature embryos of maize variety B73 were performed in the same manner as in Example 13. The inoculum was carried out using Agrobacterium strain LBA4404 into which the binary vector pJTM4596 (Example 1) and a booster vector pVGW9 were co-introduced.

Among the immature embryo samples, the count results of the number of immature embryos with which compact calluses or Type I calluses were formed on scutellum, the number of immature embryos subjected to the redifferentiation culture, and the number of immature embryos with which plants having generated PPT resistant shoots and roots were obtained are presented in Table 13.

### [Table 13]

**Table 13 Transformation results of maize to which callus induction was performed on media having various concentrations of MS basal inorganic salts**

| Agrobacterium strain | Number of immature embryos | | Callus formation efficiency rate (c:b/a) | Number of immature embryos | | Production rate of plants having re-differentiated PPT resistant shoots/ roots (c×e/d) |
|---|---|---|---|---|---|---|
| | Samples (a) | Callus formation (b) | | Redifferentiation culture samples (d) | Plants having re-differentiated PET resistant shoots/roots (e) | |
| 1×MS | 37 | 34 | 91.9% | 15 | 8 | 49.0% |
| 0.8×MS | 37 | 34 | 91.9% | 15 | 9 | 55.1% |
| 0.5×MS | 36 | 32 | 88.9% | 16 | 2 | 11.1% |
| 0.5×MS+0.5×N6 | 39 | 34 | 87.2% | 18 | 10 | 48.5% |
| 1×MS+0.5×N6 | 35 | 33 | 94.3% | 14 | 4 | 27.0% |
| 1×N6 | 39 | 23 | 59.0% | 11 | 3 | 16.0% |

The callus formation rate after performing callus induction culture for 5 weeks remained in the range of from 50% to less than 60% only with the 1×N6 medium, but the callus formation rates were 85% or higher with 1×MS, 0.8×MS, and 0.5×MS, where the MS basal inorganic salts were used at various concentrations, and 0.5×MS+0.5×N6 and 1×MS+0.5×N6 where the MS basal inorganic salts and the N6 basal inorganic salts were mixed. Part of the obtained calluses was provided for a redifferentiation culture. As a result, the plants having the re-differentiated PPT resistant shoots and roots were obtained with all the above-mentioned media.

### Example 16 Change in expression of foreign gene of 35S promoter in maize transformation

A difference in expression of the ubiquitin promoter and of the 35S promoter in maize transformation was observed using a GUS gene. Immature embryos of maize were transformed with the Agrobacterium strain pJTM3968/LBA4404 into which a binary vector pJTM3968 (Example 1) and a booster vector pVGW9 were co-introduced, and the Agrobacterium strain pSB131/LBA4404 into which a super binary vector pSB131 (Ishida et al., 1996) (NPL 15) was introduced, according to the method of Ishida et al.,(2007) (NPL 2) to produce plants having re-differentiated PPT resistant shoots and roots. The binary vector pJTM3968 (Example 1) was a vector in which the GUS gene (IGUS gene) including a castor bean catalase intron was expressed with the promoter of the ubiquitin 1 of maize (Pubi) and the first intron (Iubi). The super binary vector pSB131 was a vector in which the IGUS gene was expressed with the 35S promoter of the Cauliflower mosaic virus.

To visualize expression of the GUS gene in calluses during the selective culture, PPT resistant shoots, and the PPT resistant shoots after 2 weeks of the root culture, each tissue was immersed in a phosphate buffer solution (pH 6.8) including 1.0 mM of 5-bromo-4-chloro-3-indolyl-β-D-galactoside (X-gluc). After reacting for 24 hours at 37°C, the sites colored in blue were observed.

As a result, there was no difference in sites colored in blue between PubiIubi and P35S in the callus during the selective culture and the PPT resistant shoots. However, the PPT resistant shoots after the 2 weeks of the root culture, which were derived from the transformants using PubiIubi, displayed blue color, but the those derived from the transformants using P35S did not display blue color or displayed only a pale color.

It was demonstrated from the above-described results that P35S could express the foreign gene in the calluses of maize or shoots at the initial stage of the redifferentiation at the same level as the PubiIubi, but expression was weakened as the time lapsed from the redifferentiation of the shoots.

### Example 17 Maize transformation using ZmWox5b gene with rice actin promoter

The rice actin promoter is one of promoters generally used for overexpressing a foreign gene in maize. A ZmWox5b gene was overexpressed with the rice actin promoter to perform transformation of the arduously culturable maize variety.

The binary vector pJTM4596 of Example 1 was digested with restriction enzyme XbaI to remove and cause ring-opening of the Pubi-Iubi sequence, and a sequence composed of the rice actin promoter and a 5' untranslated region was inserted into the digested site. As a result, a binary vector having Pact-ZmWOX5bX1-Tnos::P35S-bar-T35S on T-DNA was obtained. The binary vector was introduced together with a booster vector pVGW9 into Agrobacterium strain LBA4404 by electroporation, and the resulting LBA4404 was provided for a maize transformation test. In the same manner as in Example 8, inoculation, co-culture, callus induction (5 weeks), redifferentiation culture, and root culture of immature embryos of maize variety B73 were performed.

Among the immature embryo samples provided, the count results of the number of immature embryos with which compact calluses or Type I calluses were formed on scutellum, the number of immature embryos with which PPT resistant shoots were re-differentiated, and the number of immature embryos with which PPT resistant rooting plants were obtained are presented in Table 14.

### [Table 14]

**Table 14 Results of transformation of immature embryos of maize using ZmWOX5 gene expression vector with rice actin promoter**

| Promoter | Number of immature embryos | | | | Callus formation efficiency rate (b/a) | Production efficiency rate of PPT resistant rooting plants (c/a) |
|---|---|---|---|---|---|---|
| | Sample (a) | Callus formation (b) | Generated PPT resistant shoots | PPT resistant rooting plants (c) | | |
| Pact | 133 | 64 | 18 | 0 | 48. 1% | 0.0% |

When the ZmWox5b gene was expressed with the rice actin promoter, calluses were obtained from the B73 immature embryos, but the callus formation efficiency rate was 48.1%, which was significantly lower than those of PubiIubi, P35SIcat, and PPLTP in Example 11. In addition, PPT resistant shoots were obtained, but PPT resistant rooting plants could not be obtained.

From the results described above, it was demonstrated that promoters generally used for maize transformation could not be used as the promoter used for regulating expression when transformed maize was produced using the ZmWox5b gene.

### [Sequence listing free text]

SEQ ID NO: 1 is a base sequence of ZmWOX5bX1 cDNA.
SEQ ID NO: 2 is an amino acid sequence of ZmWOX5bX1 protein.
SEQ ID NO: 3 is a base sequence of ZmWOX5bX1-GSG2A-sGFPS65T.
SEQ ID NO: 4 is a base sequence of ZmWOX2a cDNA.
SEQ ID NO: 5 is a base sequence of ZmWOX4 cDNA.
SEQ ID NO: 6 is a base sequence of ZmWOX11 cDNA.
SEQ ID NO: 7 is a base sequence of ZmWUS2 cDNA.
SEQ ID NO: 8 is a base sequence of ZmNS2 cDNA.
SEQ ID NO: 9 is a base sequence of ZmBBM2 cDNA.
SEQ ID NO: 10 is a base sequence of Pnos-ZmWUS-Tpin.
SEQ ID NO: 11 is a base sequence of TaWOX5 cDNA.
SEQ ID NO: 12 is a base sequence of OsWOX5 (synonym: OsWOX9) cDNA.
SEQ ID NO: 13 is a base sequence of ZmWOX5a cDNA.
SEQ ID NO: 14 is a base sequence of ZmWOX5b cDNA.
SEQ ID NO: 15 is an amino acid sequence of ZmWOX5b protein.
SEQ ID NO: 16 is a base sequence of P35SIcat promoter.
SEQ ID NO: 17 is a base sequence of PPLTP promoter.
SEQ ID NO: 18 is a base sequence of encoding a foot-and-mouth disease virus (FMDV)-derived 2A peptide.
SEQ ID NO: 19 is an amino acid sequence of foot-and-mouth disease virus(FMDV)-derived 2A peptide.
SEQ ID NO: 20 is a base sequence of a forward primer for amplifying from RB to LB of pLC41.
SEQ ID NO: 21 is a base sequence of a reverse primer for amplifying from RB to LB of pLC41.
SEQ ID NO: 22 is a base sequence of a forward primer for amplifying from a gentamicin resistance gene to a T-DNA.
SEQ ID NO: 23 is a base sequence of a reverse primer for amplifying from a gentamicin resistance gene to a T-DNA.
SEQ ID NO: 24 is a base sequence of a forward primer for amplifying a spectinomycin resistance gene.
SEQ ID NO: 25 is a base sequence of a reverse primer for amplifying a spectinomycin resistance gene.

## Claims

1. A method of producing a transformed cell or plant body of maize, the method comprising:
overexpressing, in maize,
1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize; or
2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize,
under the control of a promoter including a 35S promoter of Cauliflower mosaic virus.

2. The method according to claim 1, comprising:
overexpressing, in maize,
1) the nucleic acid encoding a polypeptide including an amino acid sequence having at least 95% sequence identity with the amino acid sequence of SEQ ID NO: 2, the polypeptide having a function of promoting cell division of maize; or
2) the nucleic acid encoding a polypeptide including an amino acid sequence having at least 95% sequence identity with the amino acid sequence of SEQ ID NO: 15, the polypeptide having a function of promoting cell division of maize,
under the control of the promoter including the 35S promoter of the Cauliflower mosaic virus.

3. The method according to claim 1 or 2,
wherein the overexpressing, in maize, the nucleic acid includes culturing immature embryos of the maize on a MS medium to undergo callus induction.

4. The method according to any one of claims 1 to 3,
wherein the overexpressing, in maize, the nucleic acid includes culturing immature embryos of the maize for 2 weeks or longer and shorter than 8 weeks to undergo callus induction.

5. The method according to any one of claims 1 to 4,
wherein the overexpressing, in maize, the nucleic acid includes culturing immature embryos of the maize on a medium including proline at a concentration higher than 6.1 mM to undergo callus induction.

6. The method according to claim 1 or 2,
wherein the overexpressing, in maize, the nucleic acid includes culturing immature embryos of the maize on a MS medium for 2 weeks or longer and shorter than 8 weeks to undergo callus induction.

7. The method according to any one of claims 1 to 6,
wherein the promoter including the 35S promoter of the Cauliflower mosaic virus is
(i) a promoter having a sequence including the 35S promoter of the Cauliflower mosaic virus and a castor bean catalase intron placed downstream of the 35S promoter; or
(ii) a promoter composed of the 35S promoter of the Cauliflower mosaic virus.

8. The method according to any one of claims 1 to 7, wherein abnormality of a phenotype of the maize is reduced compared to a case where the nucleic acid is overexpressed in maize under the control of a promoter other than the promoter including the 35S promoter of the Cauliflower mosaic virus.

9. The method according to any one of claims 1 to 8,
wherein the overexpressing, in maize, the nucleic acid is not carried out by expressing BABY BOOM.

10. A nucleic acid construct, comprising:
1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize, or
2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize; and
a promoter including a 35S promoter of Cauliflower mosaic virus for producing a nucleic acid in maize.

11. A method of introducing a nucleic acid into a cell or plant body of maize, the method comprising:
introducing the nucleic acid construct according to claim 10 and a desired nucleic acid into maize.

12. The method according to claim 11,
wherein a nucleic acid encoding BABY BOOM is not introduced into the maize.

13. A nucleic acid construct, comprising:
1) a nucleic acid encoding an amino acid sequence of SEQ ID NO: 2, or a nucleic acid encoding a polypeptide including an amino acid sequence having at least 95% sequence identity with the amino acid sequence of SEQ ID NO: 2, the polypeptide having a function of promoting cell division of maize, or
2) a nucleic acid encoding an amino acid sequence of SEQ ID NO: 15, or a nucleic acid encoding a polypeptide including an amino acid sequence having at least 95% sequence identity with the amino acid sequence of SEQ ID NO: 15, the polypeptide having a function of promoting cell division of maize;
a promoter including a 35S promoter of Cauliflower mosaic virus for producing a nucleic acid in maize; and
a desired nucleic acid.

14. The nucleic acid construct according to claim 13,
wherein 1) the nucleic acid encoding an amino acid sequence of SEQ ID NO: 2, or the nucleic acid encoding a polypeptide including an amino acid sequence having at least 95% sequence identity with the amino acid sequence of SEQ ID NO: 2 and having the function of promoting cell division of maize, or
2) the nucleic acid encoding an amino acid sequence of SEQ ID NO: 15, or the nucleic acid encoding a polypeptide including an amino acid sequence having at least 95% sequence identity with the amino acid sequence of SEQ ID NO: 15 and having the function of promoting cell division of maize; and
the desired nucleic acid
are directly bonded or bonded via a linker.

15. A method of introducing a nucleic acid into a cell or plant body of maize, the method comprising:
introducing the nucleic acid construct according to claim 13 or 14 into maize.
